Europäisches Patentamt

European Patent Office

Office européen des brevets

(19)

(11) Publication number: **0 202 017 B1**

# EUROPEAN PATENT SPECIFICATION

(45) Date of publication of patent specification: **16.10.91**   (51) Int. Cl.⁵: **A61K 9/50**

(21) Application number: **86302590.4**

(22) Date of filing: **08.04.86**

(54) **Artificial microcompartmentalised structures and process for the manufacture thereof.**

(30) Priority: **08.04.85 IL 74838**
     **28.01.86 IL 77724**

(43) Date of publication of application:
     **20.11.86 Bulletin 86/47**

(45) Publication of the grant of the patent:
     **16.10.91 Bulletin 91/42**

(84) Designated Contracting States:
     **AT BE CH DE FR GB IT LI LU NL SE**

(73) Proprietor: **Rafa Laboratories Ltd.**
     **P.O. Box 405**
     **Jerusalem 91003(IL)**

(72) Inventor: **Ben-Sasson, Shmuel**
     **Kiryat Moshe 10**
     **Jerusalem(IL)**

(74) Representative: **Crampton, Keith John Allen et al**
     **D. YOUNG & CO. 10 Staple Inn**
     **London WC1V 7RD(GB)**

(56) References cited:

**Chemical Abstracts, vol. 99, no.5, August 1,1983, page 287, ref.no.35648p; Columbus, Ohio, US, G.A. Weisman: "Fractionation of Triton x-100 solubilized pigeon erytrocyte membrane and reconstruction into vesicles with ATP dependent calcium transport activity**

**Chemical Abstracts, vol. 101, no. 24, December 10, 1984, pages 314-315, ref. no. 216396k; Columbus, Ohio, US & JP-A-59 157 033 (H. Tsuchida) 06-09- 1984**

Journal of the American Chemical Society, vo. 106, no. 12, June 13, 1984, pages 3613-3623; Am.Chem.Soc., US Y. Murakami et al.: "Characterization of molecular aggegates of peptide amphiphiles and kinetics of dynamic processes performed by single-walled vesicles.

Journal of Pharmaceutical Sciences, vol. 70, no. 4, April 1981, pages 358-363; Am. Pharm. Ass., US T. Ishizaka et al.: "Preparation of egg albumin microcapsules and microspheres."

Chemistry Letters, no. 10, October 1984, pages 1713-1716; Chem. Soc. of Japan, JP K. Yamada et al.: "Formation of helical super structure from singlewalled bilayers by amphiphiles with oligo-L-glutamic acid-head group.

## Description

This invention concerns a novel artificial microcompartmentalised structure and a process for the manufacture thereof. By this is meant an artificial structure, which is per se a novel form of matter, and the peripheral membrane of which has certain similarities to elements of the membranes of naturally-occurring biological cells.

Biological cell membranes consist of a continuous bilayer of lipid molecules, composed principally of phospholipids, cholesterol and glycolipids. This bilayer may be regarded as a solvent for membrane proteins, including glycoproteins, which are embedded therein. It is known that the specific functions of the membranes are largely caried out by these proteins, which may comprise some 25 to 75% of the membrane mass: "The Plasma Membrane", in "Molecular Biology of the Cell", pp.225 et seq., Alberts et al, Garland Publishing Inc.,1983.

Chang in 1956 commenced a study of "artificial cells", which consisted of a spherical ultrathin polymer membrane, enclosing a microdroplet of haemaglobin and enzymes from haemolysate. Proteins, other enzymes and further materials could also be incorporated in the enclosure. It was originally intended that such artificial cells might be used for possible replacement or supplement of deficient cell functions. The synthetic membranes, which could be made of, for example, cellulose nitrate polymer, nylon or protein cross-linked with nylon, were formed at the interface of the aqueous droplets of a water in oil emulsion: see "Artificial Cells", Chang, in Publn. No. 828, American Lecture Series, publ. Charles C. Thomas, 1972).

The membranes in the foregoing "artificial cells", however, bear little structural resemblance to naturally occurring biological membranes or their constituents, since in the former case, the membrane consists virtually of a continuous molecular structure held together by interfacial layering and/or by covalent bonds, and it has been remarked that, with regard to such "artificial cells", problems encountered with the use of synthetic materials include the inability of the body to biodegrade many of the materials used and a failure to produce capsules small enough to clear the microcirculation: (Pitt et al, Biochemical Pharmacology, 22: 3359, 1983).

By contrast, it has been demonstrated that the assembly of a biological cell membrane (for example) takes place as a thermodynamic process in which each relevant molecule searches for its state of lowest chemical potential, and this leads to the hydrophobic effect. What this means in practice is that hydrophobic moieties in the relevant molecules are repelled by an aqueous medium and are instead attracted to each other so as to form relatively weak bonds. Moreover, unlike synthetic membranes which are made from molecular layers aligned parallel to the surface, the long axis of the amphipathic linear polymers in biological membranes is orientated perpendicularly to the membrane plane. Therefore, while in the former, the width of the wall depends on the amount of added polymer, the width of biological membranes is self-limited. The hydrophobic affect accounts for the formation of micelles from e.g. detergent molecules, wherein the hydrophobic hydrocarbon chains are repelled by water and join in the centre of a star-like structure, of which the many points are represented by the hydrophilic polar terminal groups. As will be seen, the cell-like structures of the present invention are not to be identified with micelles, since the former have a cavity in which substances may be enclosed.

This hydrophobic effect also accounts for liposome structure of phospholipids in water, in which a concentric double layer of phospholipids of spherical cell-like configuration is formed, the polar groups in the inner layer of phospholipids pointing towards the internal aqueous medium and the polar groups in the outer layer of phospholipids pointing outwards towards the bulk of the aqueous medium, while the non-polar hydrophobic portions in each molecule are orientated towards each other in the spherical annulus: "The Hydrophobic Effect and the Organization of Living Matter", Tanford, Science, 200: 1012-18, 1978, and Alberts et al, loc cit. It may be noted that liposomes are relatively delicate structures. For example, they can be disrupted by mild detergents or even by exposure to some plasma constituents: see "Liposomes in Biological Systems", Gregordiadis and Allison, Eds., publ. John Wiley and Sons, 1980, pages 179-209, whereas the membranes of the microcompartments of the present invention are stable under these conditions.

US-A-4285862 describes a substantially undenatured and lipid-free amorphous protein isolate, prepared by settling an aqueous dispersion of protein micelles consisting of homogenous amphiphilic protein moieties, the dispesion having been obtained from a protein, usually of plant origin, by a salting-in and dilution-out procedure, using (mainly) NaCl, $CaCl_2$ or $NAH_2PO_4$.

The microcompartments of the invention are also to be distinguished from known industrial microcapsules, not only on the basis of molecular structure, but by their properties also. With regard to the latter, it has been stated that "in all cases, the enclosing wall of these microcapsules are made as impermeable to external and internal molecules as possible, and the enclosed material can act only when the microcapsule wall is disrupted releasing the enclosed material": see "Microencapsulation: Processes and Applications", J.. Vandegaer, Ed., Plenum Press, 1974, page 95.

The usefulness in membrane research of a number of organic solvents has been reviewed in the literature. It was noted inter alia that while n-butanol compared favourably with a series of alcohols as regards being both a good extractant and least damaging to the proteins extracted, it nevertheless led to difficulties as regards subsequent aggregation of the proteins extracted. This review also noted the lack of methods for the separation and isolation of amphipathic biopolymers and in particular of membrane proteins: "The Use of Organic Solvents in Membrane Research," Zahler and Niggli, pp. 1-50 of 'Methods in Membrane Biology' Vol.8, Plenum Press, 1977. In this context, it might be noted that n-butanol, which was a particularly useful solvent extractant for enzymes and other proteins from lipoprotein complexes has a water-solubility which varies inversely with the temperature.

In an earlier article on the extraction of enzymes from animal tissues (Morton, Methods in Enzymology, I:25-41, 1955), a number of interesting features had been remarked upon. Insofar as relates to the background of the subject of the present invention, these were that there is a notable "salting-in" effect of various salts on the aqueous extraction of proteins, the solvent power of anions diminishing in the order $P_2O_7 > B_4O_7 > PO_4 > SCN > HCO_3 > I > Cl$ at neutral or alkaline pH and citrate > acetate at acid pH.

Hatefi and Hanstein (Proc. Nat. Acad. Sci. U.S. 62:1129-36, 1969) have found that chaotropic ions, which favour the transfer of apolar groups to water, are effective for resolving membranes and multicomponent enzymes and for increasing the water solubility of particulate proteins and nonelectrolytes. This effect was said to be due to the ions in question changing the structure of water in the direction of greater disorder. It was found that the thiocyanate ion had the most dissociating and solubilizing effect on a variety of biopolymers, while sulphate, fluoride and acetate have the least effect. The same authors have further discussed the destabilization of membranes with chaotropic ions in Methods in Enzymology, XXXI:770-90, 1974. They found a decreasing order of potency in most cases as follows: $CBrCOO > CClCCO > SCN > guamidinium > I > ClO_4 > CHCl_2COO > NO_3 > Br > CF_3COO > CH_2ClCOO$, and also pointed out that (inter alia) chaotropes are potent denaturants, as well as initiating membrane lipid auto-oxidation which has vast destructive potential, because proteins are modified by auto-oxidation products, e.g. malonaldehyde.

Chemistry Letters No. 10 (10-84) pages 1713-1716 discloses the behaviour of amphiphiles with an oligo-L-Glutamic acid-head group. $2C_{16}Glu_{14}$ is said to form pre-vesicles. The formation of vesicles or lamellae by synthetic macromolecular molecules comprising a hydrophobic double chain bound to polyethyleneglycol head is also referred to at page 1714, line 10-12. However, the dialkyl amphiphiles that form bilayer membranes in this document are of molecular weight below 3000 and not classifiable as proteinaceous. They are hydrophilic and can enclose only hydrophilic substances.

J. Phar. Sci. Vol. 70, No. 4 (04-81) pages 358-363 discloses egg albumin micro-capsules which may also contain magnetite, doxorubicin hydrochloride or phenacetin. There is no peripheral membrane in such microcapsules.

Chemical Abstracts 99 35648p suggests that pigeon erythrocyte membrane is fractionated with Triton X-100 and then these fractions are reconstituted into vesicles. The fact that these vesicles exhibit ATP-dependent $Ca^{++}$ transport activity can be ascribed only to the presence of membrane proteins possessing that activity. An essential feature is the addition of liposomes for the reconstitution of enzyme activity from the column fractions. However, liposomes are spherical vesicles formed from relatively low molecular weight lipids which form bilayers; they are themselves of comparatively low molecular weight.

The invention accordingly provides a semipermeable microcompartment which is artificially prepared by reassembly of proteinaceous substances and which is defined by a peripheral membrane which consists substantially of a layer of macromolecules each of which comprises a relatively hydrophilic moiety and a relatively hydrophobic moiety and wherein the majority of such macromolecules forming the membrane are disposed with their relatively hydrophilic moities orientated outwardly from the microcompartment and their relatively hydrophobic moieties orientated inwardly towards the interior of the microcompartment. In other words, the long axis of the amphipathetic linear polymers is aligned perpendicularly to the membrane plane, and therefore the membrane width is self-limited. It is frequently found, when the microcompartments are prepared by the method described hereinafter, that substantially all of the macromolecules in the membrane layer are disposed with their relatively hydrophilic and relatively hydrophobic moieties orientated as aforesaid.

The microcompartment of the present invention, while agreeing in principle with Tanford's concept of the Hydrophobic Effect, is yet completely different in structure from any of the amphipathic structures hitherto described in the literature.

At this point it may be noted that Alberts et al (loc cit, pages 127-8), at the end of a discussion on limits to macromolecule self-assembly, states that "..... not all structures in the cell are capable of spontaneous reassembly if they are dissociated into their component parts.". Nevertheless, in the microcompartment of the present invention, the major part of the macromolecular content of a naturally occurring biological cell membrane is reconstructed so as to form the peripheral membrane fabric of the microcompartment, notwithstanding the doubts that may be thought to have been cast on this possibility by the views just mentioned.

In accordance with preferred aspects of the present invention, the microcompartments are not only semipermeable (unlike industrial microcapsules), but also may be composed of macromolecular units, in many case proteins, held together by non-covalent bonds (unlike "artificial cells", where proteins have to be either crosslinked with, or adsorbed onto the surface of, synthetic polymers), and have a capacity for enclosing various materials within a central cavity (unlike micelles, which have no such cavity).

In the preparation of the microcompartments of the present invention, the fabric material is not limited to proteinaceous or other naturally occurring substances.

The microcompartments of the invention are prepared by reacting suitable macromolecular substances as hereinafter defined with a mixture comprising an organic solvent having prescribed properties and a chaotropic ion.

It must be regarded as surprising that having regard to what is known in accordance with the Hydrophobic Effect, and the apparent limits to cell macromolecule reassembly mentioned above, and moreover regarding also what is known in the literature about the structure in aqueous medium of amphipathic molecules, that is to say that they are known to form either micelles or bilayers especially with reference to the liposomes, that it was possible to obtain the orientated cell-like structures in accordance with the invention. This is even more surprising when it is realized that in US Patent Specification US-A-4285862, the ordered structure of the substantially lipid-free proteins, before settling, was that of micelles.

It is also a substantial departure from what is known in the literature to utilize a mixture of chaotropic ions and organic solvent, since although methods of isolating proteins and enzymes have been under investigation for many years, no such combination has been suggested as a viable proposition. Indeed, as regards the preferred mixture of thiocyanate ion and n-butanol, the literature may be thought to teach away from such a possibility, insofar as each method taken separately has in addition to an area of usefulness, balancing inbuilt disadvantages, as has been described above.

In the course of the process of the invention, when the microcompartments are in the process of becoming organized, they are generally spherical in shape, with the hydrophobic medium occupying the control cavity. If the medium contains other relatively insoluble suspended materials, or even other dissolved materials, these will also be found present, dissolved and/or suspended in the spherical cavity. As hydrophobic medium is removed, as for example by dialysis, the spherical shape may suffer a progressive collapse, whether or not further suspended or dissolved materials are present. If, however, such further materials are present within the cavity, they will tend to be concentrated in a smaller volume than the original microcompartment, with the consequence that the shape of such a microcompartment will tend towards that of a sphere inserted to fit into the central space of an annular disc, because the latter is formed by collapse of the outer edges of the original generally spherical periphery, where such edges do not enclose other matter which was originally suspended and/or dissolved in the medium.

It has been found that the microcompartments of the invention have unique properties, and in particular they are stable, semi-permeable and, as has already been intimated, they have an interesting and useful ability to entrap other molecules.

Unlike liposomes, the microcompartments of the invention are very stable. Thus, they are resistant to mild detergents and can be preserved in a lyophilized state, so that on resuspension they can resume their full activity, while retaining the selective permeability of the peripheral fabric.

The dimensions of the microcompartments of the invention can vary over a wide range. Thus, their overall diameter can be in the range of from about 0.1 to about 100 microns, and the thickness of the peripheral membrane will generally fall within the range of from about 100Å to about 1000Å (0.01μm to 0.1μm). The width of the basic membrane unit is self-limited and it depends on the nature of, and not the amount of, the constituents.

As to the materials from which the microcompartment fabric may be constructed, these may be selected from a wide range, provided that the relevant macromolecules comprise in their structure, relatively hydrophobic and relatively hydrophilic portions.

In one embodiment of the invention, the macromolecules forming the peripheral fabric of the microcompartments comprise protein molecules. In another embodiment of the invention, the macromolecules comprise glycoprotein molecules. In yet a further embodiment of the invention, the macromolecules comprise at least two of protein, glycolipid and glycoprotein molecules. Proteinaceous starting materials may result from the products of biotechnology ,as for example, such products which result from the manipulation of bacteria to produce proteins, e.g. viral antigenic determinants or cell surface receptors or an antibody. Alternatively, however, and this is a more preferred source of the fabric of the microcompartments, the proteins and/or glycolipids and/or glycoproteins may be present in the membranes of naturally occurring cells, from which they are extracted and orientated in accordance with the process of the present invention. When this more preferred embodiment of the invention is carried out, it is found that the phospholipid content of the original cell walls is now contained within the cavity of the microcompartments. It is to be stressed, however, that the invention equally relates to and comprises the microcompartments herein described, when the peripheral fabric is formed for example only of proteins, or only of glycoproteins, as well as those in which the peripheral fabric is derived from any two or all three of proteins, glycolipids and glycoproteins.

When naturally occurring cell membranes are utilized as a source of the microcompartment peripheral fabric, these may originate as, for example, the membranes of red blood cells, various eukaryotic cells or cells of prokaryotes. It has been found in practice that as much as about 90% of the original proteinaceous materials of a naturally occurring cell membrane can be reconstituted by the process of the invention.

As has already been indicated, the fabric of the microcompartments of the invention may be constituted of any suitable materials, like common sources rich in proteins such as casein, egg white, whole blood, or its constituents such as albumin, haemoglobin, fibrinogen, immunoglobulins, or, as for example, those wherein the macromolecules comprise synthetic polymer molecules. Particularly suitable synthetic polymers are those which result from the attachment of a relatively hydrophilic polymer to a relatively hydrophobic residue.

It should be noted that the raw materials of the peripheral fabric are not restricted to insoluble macromolecules only. For example, it has been found that some of what are normally considered as soluble proteins, are also able to assume a membranous structure, in accordance with the present invention.

A particularly useful property of the microcompartments of the invention is that, in the course of the process for preparing them, they form around a great variety of insoluble materials, which are thereby enclosed by, and contained within the cavity of, the microcompartments.

For example, magnetic particles suspended in the preparation medium become entrapped in the cavity, thus in one aspect giving rise to a useful analytical tool, in another aspect permitting gene transfer by enclosing additionally a DNA in the microcompartment and using magnetic force to enable this to penetrate a cell wall, and in yet another aspect offering a convenient and flexible technique by which the microcompartments may be isolated.

This property of enclosure or entrapment may also be applied to, for example, (a) heterogenous catalysis - by generating a mediator layer around particulate catalysts to facilitate the interaction of soluble reactants via a gradual change in the hydrophobicity of the micro-environment, or (b) standards for cell diagnosis, in which polystyrene beads are enwrapped by the microcompartments, the membranes of which contain cell-surface markers of specific cell populations.

Microcompartments may also be constructed of membranes which contain receptors for a specific ligand, e.g. a hormone. In this case the microcompompartments may be used for (a) assay of ligand, e.g. the hormone level in the serum, by competition with a fixed amount of labeled hormone, or (b) immunizing with receptor-containing membranes in order to elicit an auto-immune response against a receptor, so that the antibodies will be produced which either (i) stimulate the cells by binding to the receptor, or (ii) inhibit a hormonal action by masking the receptor. In this manner, for example, combined vaccines can be constructed to enhance immunization, by incorporating adjuvants into microcompartments, the membranes of which present antigens. In another aspect, microcompartments which enclose antibodies may be used for immunoassay of hormones or drugs.

In a further embodiment of the invention, the microcompartments may be used to enclose, i.e. concentrate or otherwise include, proteinaceous substances, whether soluble or insoluble, and whether or not such proteinaceous substances have been incorporated into the membrane itself. These proteinaceous substances may be naturally occurring, e.g. they may represent those portions originally forming part of a natural cell. The proteinaceous substances may alternatively have been artificially made by synthesis, or by a biotechnological method, and they may again represent those portions of protein produced by such methods. The biotechnological method may be for example that utilizing manipulation of bacteria as has been indicated hereinbefore.

Yet a further embodiment of the invention is that wherein the membrane of the microcompartment encloses an enzyme or an apo-enzyme. Many applications of this embodiment of the invention will suggest themselves to those skilled in the art, as for example, enzyme-based diagnosis, therapy or commodity production. Certain of these methods will require than an apo-enzyme be enclosed in the microcompartment initially, and that a coenzyme be incorporated at a later stage by transfer through the semipermeable membrane of the microcompartment. Merely by way of illustration only, the enzyme or apo-enzyme to be enclosed in the microcompartment may be selected from the group consisting of alkaline phosphatase, asparaginase, catalase, cholesterol oxidase, cholinesterase, apo-glucoseoxidase, glucoseoxidase, peroxidase, urease, glycerolphosphate oxidase and uricase.

In a further useful embodiment of the invention, the peripheral membrane of the microcompartments is fabricated of pharmaceutically compatible materials and encloses a pharmacologically active substance. This embodiment provides an advantageous method of drug delivery, with possible targeting aimed at a desired site. A wide range of pharmaceutically active substances may be utilized. They may for example have antibacterial, antifungal, antiparasitic, anti-inflammatory, anticancer, central or peripheral nervous system, analgesic, local anesthetic, narcotic or antidepressant activity, or they may have properties useful in the treatment of heart disease.

Alternatively, the pharmacologically active substances may have immunomodulating, e.g. immunostimulating or immunosupressive activity; examples of immunomodulating compounds are leukotrienes and interleukins, while Cyclosporin A is a particular example of a compound with immunosuppressive activity. In one embodiment, the microcompartments may contain a vaccine.

It will be apparent that the microcompartments which contain a pharmacologically active substance, as set out in the foregoing description, may form part of a pharmaceutical composition comprising also a suitable carrier or diluent, and such compositions also form part of the present invention; at least part of the carrier or diluent may be enclosed in the microcompartments together with the one or more pharmacologically active substances. Such pharmaceutical compositions are preferably constituted in a form suitable for administration by inhalation or insufflation, or for oral, paranteral or rectal administration, and they may be in unit dosage form. As a non-limiting example of such pharmaceutical compositions, it may be mentioned that e.g. an anti-inflammatory steroid enclosed in microcompartments of the invention and suspended in a medium suitable for parenteral administration may be advantageously targeted at the site of the inflammation.

This application of the invention, i.e. targeting, may be explained as follows. The limitations of the parenteral route of administration are generally well known. Injected vesicles will be taken up, exclusively, by the reticuloendothelial system. This phenomenon can however be considered as a great advantage, if it desired to deliver drugs specifically into macrophages. Glucocorticoids are excellent candidates for such targeting. They can be considered as a general attenuator of macrophage functions and prove to be the drugs of choice for treating inflammation. Steroid treatment suffers however, as is well known, the disadvantage of side-effects, due to the fact that, the steroids being small hydrophobic molecules, injection results in an even distribution in all tissues. However, their entrapment inside the microcompartments of the invention can increase their therapeutic index by several orders of magnitude. Other therapeutic agents which may similarly be targeted into macrophages are:

activators of macrophage functions like glucoseoxidase, which produces hydrogen peroxide, or the known macrophage activating factor; and

drugs against intracellular parasites which reside in the reticuloendothelial system, e.g. Amphotericin B against fungal infections.

In many instances, conventional administraton of a free drug may cause side effects which severely limit its practical scope of application. For example, the above mentioned Amphotericin B is highly nephrotoxic. Application in practice of the immunosuppressive drug Cyclosporin A is similarly limited by its nephrotoxicity. Likewise, undesirable side effects are shown by anticancer drugs when conventionally administered in the free form; for example, cisplatin is both nephrotoxic and neurotoxic, while doxorubicin is cardiotoxic.

In such cases, the entrapment of the drug in accordance with the present invention should (for example in the case of nephrotoxicity) assist it to avoid glomerular filtration and decrease its availability to vulnerable tissues. The differential distribution obtained by microcompartmentalization can therefore lead to a decrease in the toxicity and an increase in the efficacy of many pharmacologically active materials.

In a further embodiment of the invention, the peripheral membrane of the microcompartments may be composed of edible materials and may enclose a foodstuff or a substance compatible therewith. Quite apart from the fact that the membrane may be composed of, and may also contain, nutritious proteinaceous materials, its semipermeable properties have this particular advantage, namely that volatile substances such as food flavorings and essential oils may be released slowly over a long period. Of course, the microcompartments of the invention may enclose not only food flavorings and essential oils, but also one or more hydrophobic substances in general.

As indicated, the property of slow release by the semipermeable peripheral membrane of the microcompartments is not limited to the slow release of volatile food ingredients. It may well be desirable on other grounds, for example, that substances with growth control activity, or herbicidal, fungicidal, acaricidal or insecticidal activity, be released slowly to their environments, and the invention comprises the microcompartments containing substances with such activity, also. Indeed, it is envisaged that the semipermeable membrane property of slow release of volatile substances would find an especially useful application in the field of the biological control of insects by the use of pheremones, the utility of which is at the present time limited by their volatile character.

Yet a further useful embodiment of the invention based on the slow release of volatile substances by the semipermeable peripheral membrane will be in the field of perfume compositions. Thus, the invention also comprises microcompartments as defined herein, wherein the peripheral membrane encloses a perfume composition or perfume concentrate composition.

A still further consequence of the selective permeability of the peripheral membrane is that the membrane may enclose one or more reactants of relatively high molecular weight and admit therethrough a reactant of relatively low molecular weight. It will be evident that the reaction can take place in this micro-environment in a very high concentration of reactants which would generally not be attainable in the usual macro-environment. Thus, in accordance with the invention, there is also provided a method of effecting a chemical reaction wherein a reactant macromolecular substance of molecular weight of at least about 6000 which is enclosed in a microcompartment as defined herein, is reacted in a suitable medium with a substance of molecular weight of up to about 1000.

As has already been stated, the membrane of the microcompartments of the invention consists of a layer of macromolecules orientated in a particular manner. While the present invention is of course not limited by any theory as to the more detailed structure of this layer, it is nevertheless believed that at least in certain cases, the layer may be monomolecular.

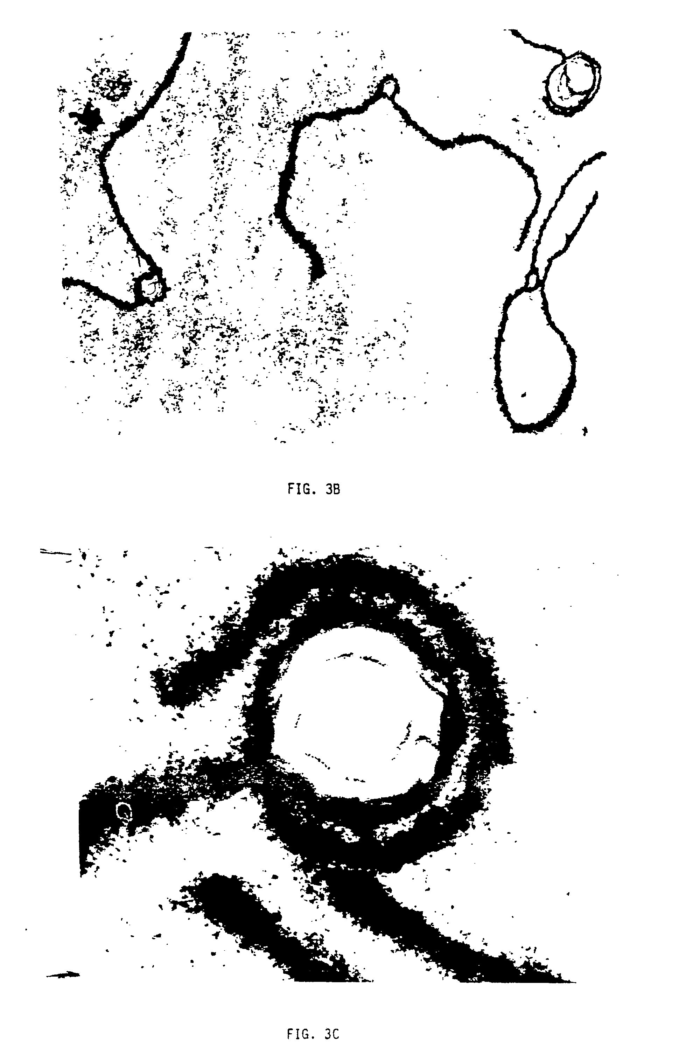

Thus, for example, electron micrographs of the reconstructed membranes of microcompartments, from either human red blood corpuscle ghosts or mouse tumor cells, prepared in accordance with the present invention, revealed a structure about 200Å(0.02μm) wide, see e.g. Figs. 3c and 3d. This finding accords with the dimensions of the native cell coat generated by membrane macromolecules (for a review, see Luft, "The Structure and Properties of the Cell Surface Coat," Int. Rev. Cytol., 45: 291-382, 1976).

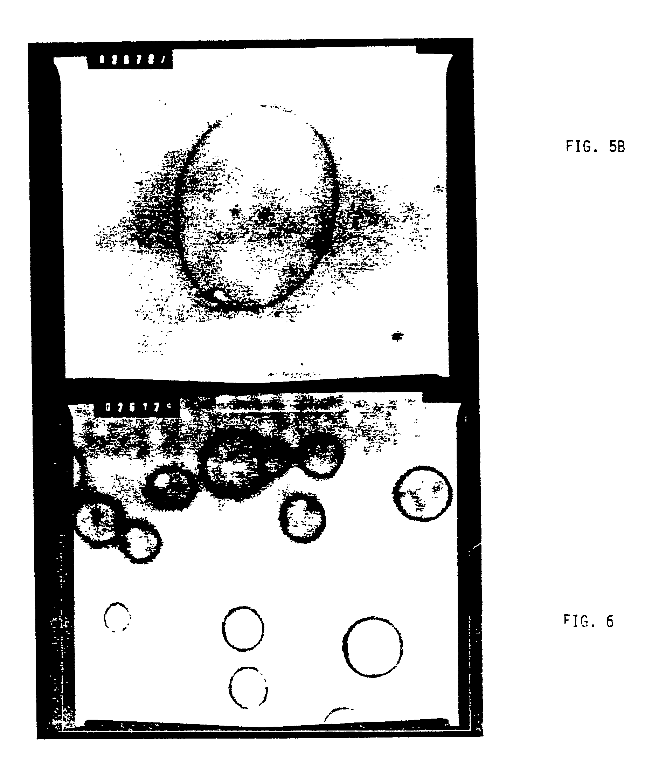

Similarly, reconstructed membranes prepared from casein have a width of about 100Å(0.01μm), see Fig. 5b, which agrees closely with the estimated length of the individual casein molecules (Waugh et al, Biochemistry, 9: 786-95, 1970).

There is also provided in accordance with the present invention, a process for preparing the microcompartments as defined herein, which comprises exposing a macromolecular substance, which contains a relatively hydrophilic moiety and a relatively hydrophobic moiety, to the solubilization action of a homogeneous mixture of chaotropic ions in water and a dialyzable organic solvent which is not completely miscible with water, if necessary or desired subjecting the mixture to filtration or centrifugation, forming microglobules containing the microcompartment precursors, and dialyzing out the organic solvent and the chaotropic ions.

It may be that in certain cases the macromolecular substance is completely dissolved by the solubilization action and the optional filtration or centrifugation step is unnecessary. When there remains insoluble matter in the mixture, however, it may be dealt with in the following manner. Where it is not desired that the microcompartments enclose insoluble materials present in the mixture, the latter should be filtered or centrifuged from insoluble matter before forming the microglobules as aforesaid, otherwise the filtration or centrifugation step may be omitted. The filtration or centrifugation step is of course carried out when it is desired either that the microcompartments enclose soluble matter present in the mixture, or that they enclose soluble matter to be added before formation of the microglobules. The filtration or centrifugation step is also obviously carried out when it is desired that the microcompartments enclose insoluble materials other than those present in the mixture; in this instance, such insoluble materials are conveniently added after the filtration or centrifugation step.

In the preparative process, the dialysis step may be effected in a dialysis bag. It is preferred that prior to dialysis the mixture is subjected to vigorous agitation as e.g. by ultrasonic means, to ensure fine division of the microglobules. The organic solvent may be for example an aliphatic alcohol containing 4, 5 or 6 carbon atoms. The use of n-butanol is particularly preferred. The preferred chaotropic ions are $CCl_3COO^-$ and $^-SCN$.

In the process according to the invention, it has been found especially advantageous to use in the process substantially equal parts by volume of an aqueous solution of chaotropic ions of concentration in the range of about 20 to about 50 % w/v, and of the organic solvent.

Regarding the step in which microglobules are formed, if the organic solvent is one (such as n-butanol) the water-solubility of which decreases with a rise in temperature, then microglobule formation is effected by raising the temperature, and/or by diluting the solution with water. If on the contrary the organic solvent is one, the water-solubility of which increases with a rise in temperature, then microglobule formation is effected by lowering the temperature and/or diluting the solution with water.

It will be appreciated from the foregoing description that however the water-solubility of the organic solvent to be used in the process varies with temperature, microglobule formation may in any case be aided by diluting the solution with water. If, on the other hand, a substantially immiscible organic solvent such as n-decanol is added to the extract instead of water, forming a water-in-oil emulsion, a reversed phase microcompartment is formed in which the peripheral membrane comprises macromolecules in which the relatively hydrophobic moiety is orientated towards the exterior of the microcompartment, and the relatively hydrophilic moiety is orientated towards the interior of the microcompartment. The invention comprises also this modification of the described process.

KEY TO THE ACCOMPANYING FIGURES

Fig. 1 shows a phase-contrast micrograph of microcompartments reconstructed from HRBC ghosts, prepared according to Example I, x 450 magnification. The general shape is that of a collapsed sphere. The dark "buttons" are internally entrapped phospholipid bilayers.

Fig. 2a shows a phase-contrast micrograph of microcompartments reconstructed from EL-4 cells, prepared according to Example II, x 450 magnification. The general shape is that of a collapsed sphere. The dark "buttons" are internally entrapped phospholipid bilayers.

Fig. 2b shows a scanning electron micrograph of partly collapsed microcompartments reconstructed from EL-4 cells. prepared according to Example II, x 3750 magnification.

Fig. 3a shows a phase-contrast micrograph of microcompartments reconstructed from Ehrlich ascites (EA) cells, prepared according to Example II, x 450 magnification. The general shape is that of a collapsed sphere. The dark "buttons" are internally entrapped phospholipid bilayers.

Fig. 3b shows an electron micrograph of a cross-section through microcompartments reconstructed from EA cells, prepared according to Example II, x 29250 magnification. The structure is that of a collapsed sphere which includes internal concentric bilayers.

Figs. 3c and 3d show different cross-sections of the same microcompartments as Fig. 3b, but with x 225000 magnification. It may be noted that the width of the surface layer of the non-collapsed portion of the microcompartments is half the width of the "tails", which represent the collapsed portions of the microcompartments.

Fig. 4a shows a phase contrast micrograph of microcompartments enclosing haemoglobin, prepared from HRBC of blood group A, according to Example IV, x 450 magnification. The agglutination was induced by incubation with an anti-A antiserum.

Fig. 4b shows the same as Fig. 4a except that the incubation was carried out in the presence of anti-B antiserum.

Fig. 5a shows a phase contrast micrograph of microcompartments reconstructed from casein according to Example XII, with a small amount of entrapped haemoglobin, x 450 magnification. In this case it may be noted that the microcompartments have not collapsed.

Fig. 5b shows an electron micrograph of the preparation described in Fig. 5a, negatively stained, x 90000 magnification.

Fig. 6 shows an electron micrograph of biocompatible microcompartments enclosing corn-oil, negatively stained, prepared according to Example XIII, x 20000 magnification.

Fig. 7 shows titration curves of surface-exposed TNP residues. For further details, see Example XV.

Fig. 8a shows a phase contrast micrograph of microcompartments containing entrapped haemoglobin, prepared according to Example IV, and incubated for 30 mins. at 37° C, in the absence of proteolytic enzyme, x 450 magnification.

Fig. 8b shows the same as Fig. 8a, except that the incubation was carried out in the presence of 0.25% trypsin (see Example XVII).

The Examples which follow are given in order to illustrate, but not to limit, the present invention.

EXAMPLES

The following is a list of source materials used in the Examples:

A. Thiocyanate stock solution is obtained by dissolving 500g. of $NaSCN.2H_2O$ in 600ml. distilled water.

B. Trichloroacetate stock solution consists of a saturated aqueous solution of $CCl_3COONa$ at room temperature, pH 7.0.

C. Human Serum Albumin(HSA) stock solution is a 25% sterile solution, salt poor, for intravenous injection (Magen David Adom, Israel).

D. Human Haemoglobin stock solution: 5ml. of human blood were washed with saline and the red blood corpuscle pellet was haemolyzed with 32.5ml. cold solution of hypotonic PBS, i.e. phosphate buffered saline diluted 1: 6 with distilled water. The haemoglobin-containing supernate was collected following the sedimentation of the ghosts by high speed centrifugation.

Example I: Solubilization and reconstruction of membranes from HRBC ghosts using a mixture of thiocyanate and n-butanol.

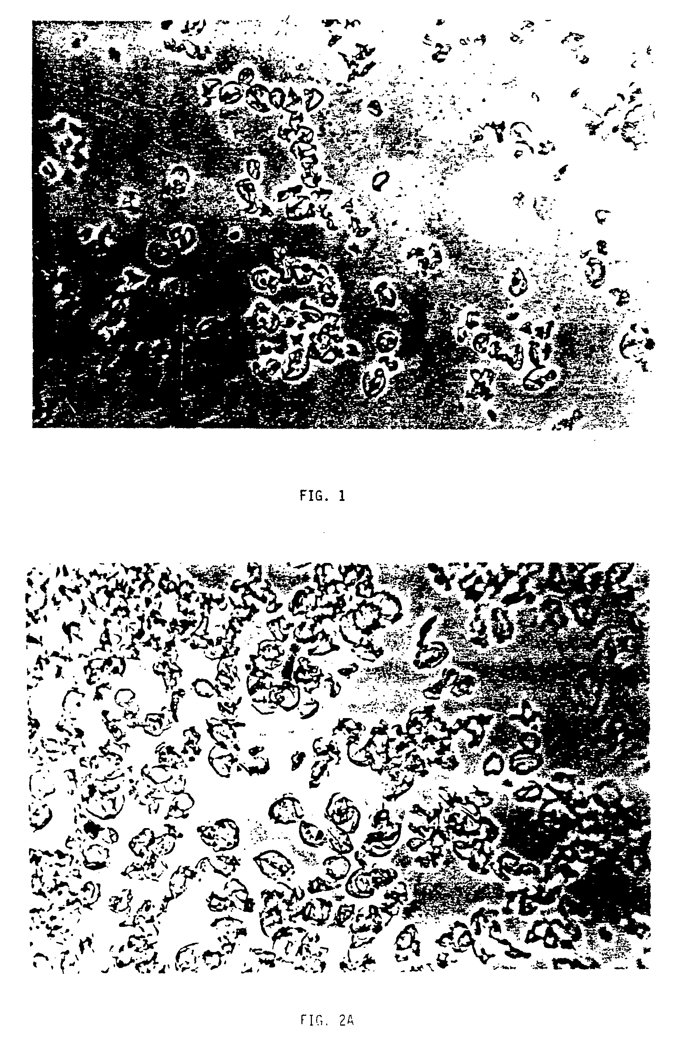

HRBC ghosts were prepared from 0.5ml. of human blood, washed twice with PBS. The HRBC pellet was resuspended twice in cold hypotonic PBS and the haemolytic supernate was discarded following high-speed centrifugation. The pellet was kept in an ice basket. These HRBC ghosts were dissolved by the addition of 5ml. of cold extraction solution composed of 1ml. thiocyanate stock solution + 1ml. n-butanol + 3ml. distilled water. After mixing in the cold for about 15 mins., the ghosts' extract was transferred to a dialysis bag. The bag was put into a 50ml. test-tube which contained water at 37°C, with the result that phase separation took place inside the bag. The test-tube with the bag were rotated at a speed of 100 r.p.m. In order to keep the contents in a constant state of emulsion. The warm distilled water was changed every 15 mins., and after four changes, the bag was transferred to a 2l. Erlenmeyer flask for dialysis against distilled water at 37°C, for several more hours. The water was changed and the dialysis was continued overnight in the cold. Both the thiocyanate and the n-butanol were readily removed by the dialysis process. As the dialysis proceeded, the hydrophobic droplets disappeared and the microcompartments formed; a phase-conrast micrograph is shown in Fig. 1.

Example II: Preparation of reconstructed membranes from nucleated cells and solid tissues.

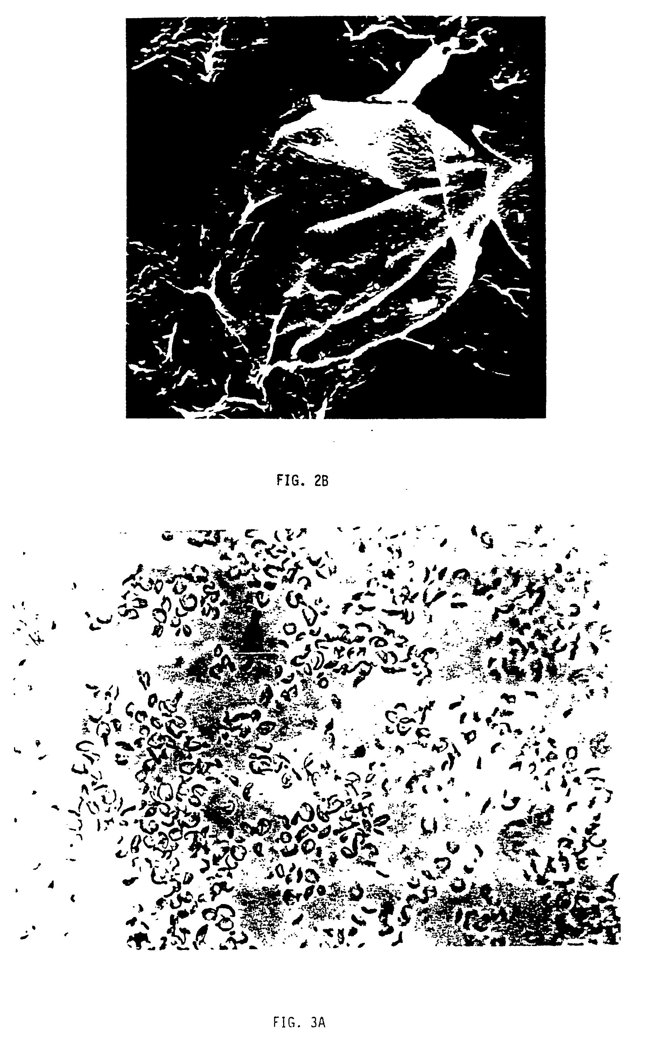

The procedure described in Example I was strictly followed, using 5 × 10⁷ cells of mouse ascitic tumor(EL-4 or Ehrlich ascites), or 5 × 10⁷ mouse spleen cells. The only difference was a removal of the nuclei, after the membrane solubilization was completed in the cold, by a slight centrifugation, also in the cold. The same procedure was applied to minced solid tissues. In all of these cases the reconstructed membranes appeared under the light microscope, like those prepared from HRBC ghosts, as illustrated in Figs. 2a, 2b and 3a to 3d.

Example III: Solubilization and reconstruction of membranes from HRBC ghosts, using a mixture of trichloroacetate and n-pentanol.

The procedure of Example I was strictly followed, except that the extraction solution was composed of 0.3ml. of n-pentanol + 1.5ml. trichloroacetate stock solution + 2.7ml. of distilled water. Another difference was that phase separation was effected in this case by the addition of 2ml. of distilled water, after 15 mins. incubation in the cold. After briefly mixing, the emulsion was transferred to a dialysis bag and the procedure was continued as described in Example I.

Example IV: Preparation of smaller reconstructed microcompartments by sonication and the concentration of haemoglobin therein.

Ghosts' pellet was prepared from 1.7ml. of human blood, solubilized by a cold mixture of 1ml. n-butanol + 1ml. thiocyanate stock solution + 3ml. of human haemoglobin stock solution. This membrane extract plus a soluble protein was submitted to ultrasonic processing by use of an MSE sonicator, model 60W, 1.7 amp intensity, according to the following schedule: 45 secs. sonication during which the mixture was warmed, phase separation took place, and a fine emulsion was created. During the subsequent 90 secs. of sonication, 9ml. of distilled water was added dropwise, and sonication was then effected for a further 45 secs. The entire suspension was transferred into a dialysis bag, and submitted to exhaustive dialysis against distilled water in the cold. The resulting microcompartments were different from those obtained in example I in that they were much smalller(approximately 2µm diameter instead of 15µm), and about 95% of the initially free haemoglobin became entrapped inside rthe microcompartments. In other words, about × 50 concentration factor of a soluble protein had occurred.

Example V: Surface expression of glycolipid antigens.

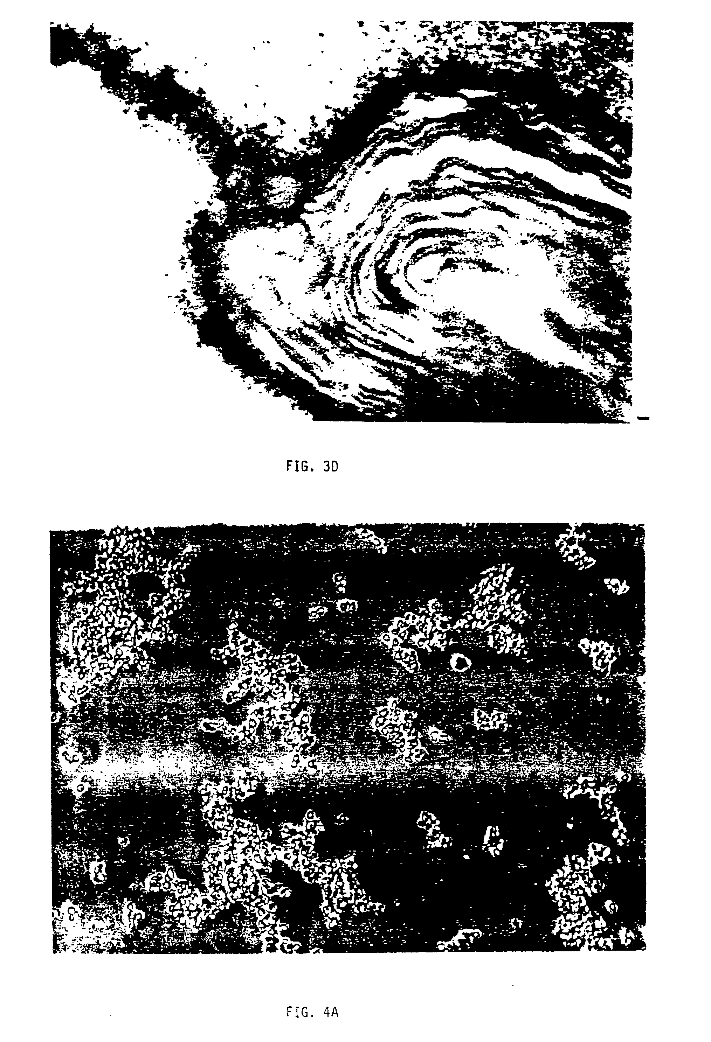

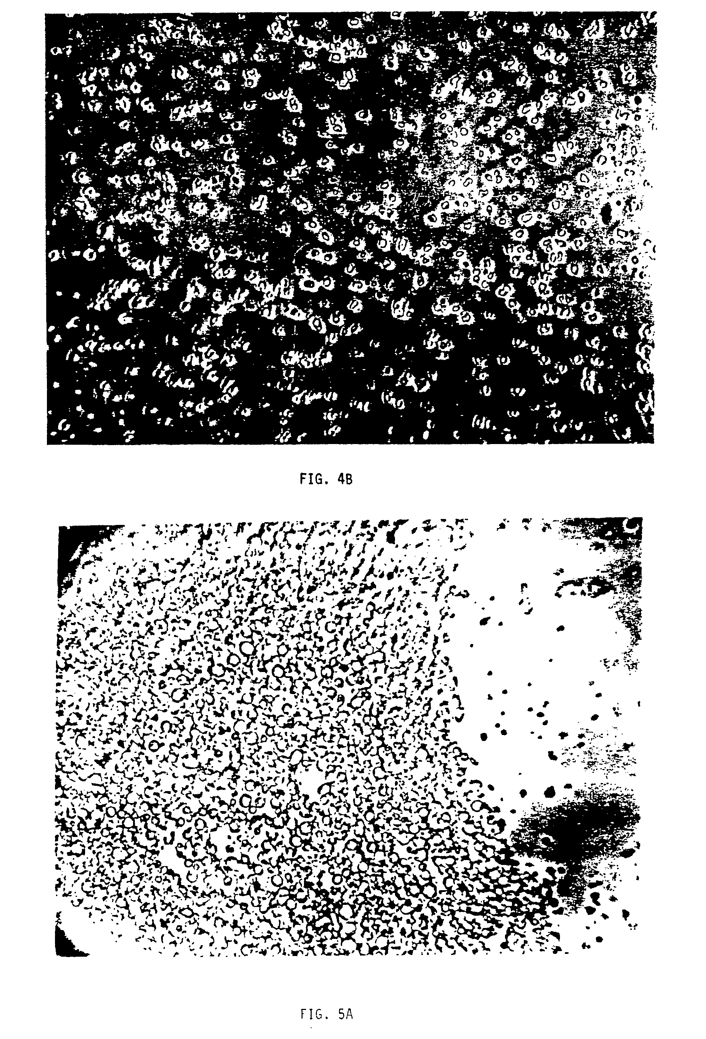

Microcompartments with peripheral reconstructed membranes prepared according to Example I or Example IV, express on the surface the same blood-group antigens as the cells of origin. E.g., membranes that were prepared from type A HRBC ghosts became agglutinated by anti-A antibodies, but not by anti-B antibodies, and vice-versa, as depicted by Figs. 4a and 4b.

Example VI: Surface expression of proteinaceous antigens.

Microcompartments with peripheral reconstructed membranes prepared from EL-4 cells (raised in C57bl mice), according to Example II, express the major histocompatibility complex(MHC) antigens on their surface, i.e. they specifically bind an anti-H-2$^b$ antiserum, but not an anti-H-2$^d$ antiserum, as determined by subsequent exposure to protein-A carrying bacteria.

Example VII: Generation of microcompartments with mosaic peripheral membranes.

Membrane extracts were prepared separately from cells of blood groups type A and type B, according to Example I, and the extracts were then mixed in the cold, prior to the reconstruction phase. The resulting membranes behave like those prepared from an AB donor, i.e. upon incubation with either anti-A or anti-B antisera and subsequent staining with a fluorescent second antibody, the microcompartment peripheral membranes were homogeneously stained on the surface.

Example VIII: Immunogenicity.

AKR/J mice were injected with either intact thymus cells from Balb/c mice, or with microcompartments having reconstructed peripheral membranes from such cells, according to Example II. The same titer of anti thy-1.2 antibodies was obtained in each case, as measured by the method of Boyse et al(Methods Med. Res., 10: 39, 1964).

Example IX: Expression of surface receptors, and the inclusion of magnetic particles.

Microcompartments with reconstructed membranes were prepared from turkey erythrocytes ghosts according to Example II. For reasons of convenience in the subsequent assay(= in the washing step), tiny magnetic particles were included in the extraction mixture [the n-butanol contained 2% w/v of magnetic iron oxide (TMO-N2325, Hercules Inc.)]. Following the reconstruction, these magnet-containing membranes were found to bind insulin specifically, like the native cells. The assay was performed according to the method described by Ginsberg et al (Endocrinology, 100: 82, 1977).

Example X: Preparation of microcompartments from egg white constituents.

1g. of freshly-separated egg white was admixed with 2ml. of n-butanol + 2ml. of thiocyanate stock solution in the cold. 5ml. of cold distilled water was then added, and the whole thoroughly mixed in the cold. Ultrasonic processing was carried out using Sonicator model W-375 (Heat Systems Ultrasonic Inc.), at step 6 of the output control setting, according to the following schedule: an initial 1 min. sonication to obtain a fine emulsion, then while continuing sonication, 18 ml. cold distilled water was added gradually over 30 secs., and the sonication continued for a further minute. The suspension was transferred to a dialysis bag, and dialysed in the cold for 2 days with a change of water each 12 hours. The product is found to comprise spherical microcompartments of 2-5μm diameter, having reconstructed peripheral membranes.

Example XI: The inclusion of oily materials within microcompartments prepared from egg white.

Microcompartments were prepared from egg white constituents according to Example X, except that before the addition of the first 5ml. of cold water, 0.1ml. of an oily material was added to the egg white extraction mixture. Either 0.1ml. of soya bean oil, or lemon grass oil diluted 1: 5 with soya bean oil were used. After being thoroughly mixed, 5ml. of cold distilled water were added, and the process was continued as described above. The resulting microcompartments, about 5u in diameter, contain the oily material as internalized oily droplets. In the case of the microcompartments containing lemon grass oil, a sustained release of the typical odour was obtained.

Example XII: Microcompartments from casein and the concentration of soluble protein therein.

Casein is known to be "very sparingly soluble in water and in nonpolar organic solvents"(The Merck Index, 10th edn., 1983, page 263). However, casein is readily soluble in the mixture of an aqueous solution of chaotropic ions plus an organic solvent. 0.1 g. of casein(essentially vitamin-free, Sigma) was dissolved in a cold mixture of 2.5ml. of thiocyanate stock solution + 2.5ml. of n-butanol. 5ml. of cold distilled water were added and the whole mixed in the cold. Ultrasonic processing was subsequently carried out exactly as described in Example X. Spherical non-collapsed microcompartments were created with a diameter of about 1u to about 10u. When 1ml. or more of the initial 5ml. distilled water was replaced with an equal volume of the human haemoglobin stock solution, at least 90% of the haemoglobin was concentrated inside the microcompartments, as determined spectrophotometrically. In accordance with the phosphoric acid residues concentrated at the hydrophilic region of the casein molecules, these reconstructed membranes were agglutinated upon the addition of calcium ions. Figs. 5a and 5b show such casein microcompartments enclosing a small amount of haemoglobin.

Example XIII: Preparation of microcompartments from biocompatible biodegradable macromolecules and entrapment of a water-insoluble steroid therein.

10mg. of (11, 17 )-17-ethylthio-9-fluoro-11-hydroxy-17-methylthioandrosta-1,4-dien-3-one were dissolved in a mixture of 5ml. of thiocyanate stock solution + 5ml. of n-butanol and stored in the cold. 2ml. of human haemoglobin stock solution were added, mixed, and followed by the addition of 4ml. of human serum albumin stock solution. 0.2 ml. of corn oil were then added and the whole thoroughly mixed. Ultrasonic processing was carried out using Sonicator model W-375 (vide supra) according to the following schedule: 50 secs. sonication at step 10 of the output control setting, during the last 20 secs of which 30ml. of cold distilled water were added. The setting was then changed to step 6 and the sonication continued for a further 50 secs., during the first 20 secs. of which 30 ml. more of cold distilled water were added. The resulting oil-containing microcompartments, diameter about 1u, was subjected to an exhaustive dialysis against distilled water in the cold.

When corn oil was omitted from the system, a precipitate of the steroid crystals developed, as the thiocyanate and the n-butanol were diluted and dialysed out. However, in the corn oil containing system, no precipitate was observed, which indicated that the steroid partitioned into the oil phase and became solubilized therein. Fig. 6 shows an electron micrograph of corn oil-containing microcompartments composed of human haemoglobin and human serum albumin.

Example XIV: Recovery of the native membrane proteins and their preserved orientation in the reconstructed microcompartments.

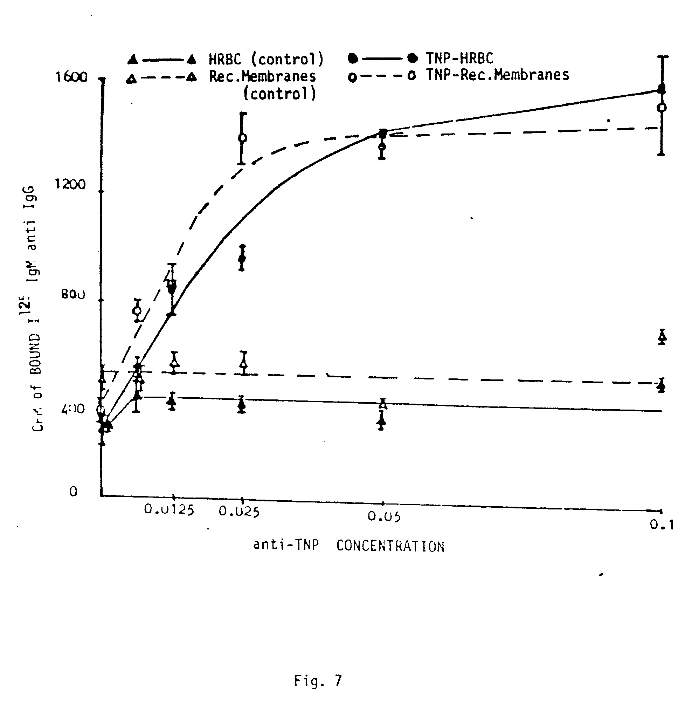

$4 \times 10^9$ fresh HRBC were washed well with PBS and incubated with 1mM trinitrobenzenesulfonate (TNBS) for 30 minutes o at 37 C. Under such conditions, trinitrophenyl (TNP) residues become covalently bound to exposed amino and sulfhydryl groups of surface proteins, while intracellular penetration of the reagent is negligible. Since the entire population of membrane proteins is modified by TNP, it can be considered as a general protein marker (Bonsall and Hunt, Biochim. Biophys. Acta 249: 281, 1971). The cells were then washed ($\times$ 3) with PBS and divided into two equal parts. Quantification of externally exposed TNP residues was then performed on the intact cells of the first group, according to the method described below. From the cells of the second group, ghosts were prepared, solubilized and processed according to Example I. The reconstructed membranes which are impermeable to macromolecules like IgG and IgM were submitted to the same TNP quantification. In addition, control cells and control membranes were prepared from HRBC that underwent the same treatment except that TNBS was omitted. Quantification of surface-exposed TNP residues was carried out as follows: limited amounts of cells or membranes were incubated in the presence of increased concentrations of rabbit anti-TNP serum for 1 hr. After being washed ($\times$ 5), the preparations were incubated with 125 monoclonal I -labeled IgM and IgG antibodies (for details see Steinitz et al, J. Immun. Methods, 54: 273, 1982) for 1 hour, washed again ($\times$ 5) and the bound radioactivity was counted. The results are depicted in Fig. 7. It can be seen that at least 90% of the originally labeled proteins are restored and have become properly orientated in their membranes of the reconstructed microcompartments.

Example XV: The reconstructed membranes are selectively permeable.

It was already evident from the studies with soluble proteins on the one hand, and an investigation of small molecular weight dye molecules on the other hand, that the reconstructed membranes are selectively permeable. Insulin molecules (MW ~6000) can be entrapped and retained by the reconstructed membranes of the microcompartments, while Evans Blue, a dye of MW ~1000, is freely permeable. This particular dye is of special interest since it has a high affinity to proteins. In order to quantify the phenomenon further, haemoglobin-containing reconstructed microcompartments were prepared according to Example IV. Various concentrations of Evans Blue were added either prior to the reconstruction, or after the process was completed. In the latter case the microcompartments were allowed to incubate with the dye for 24 hours. Both preparations were then washed thoroughly, the entrapped dye was released by treatment with 1% sodium dodecylsulfate, and determined spectrophotometrically (605 nm). No difference was found between the two systems. i.e. the dye has free access to the entrapped protein.

Example XVI: Sensitivity towards proteolytic enzymes.

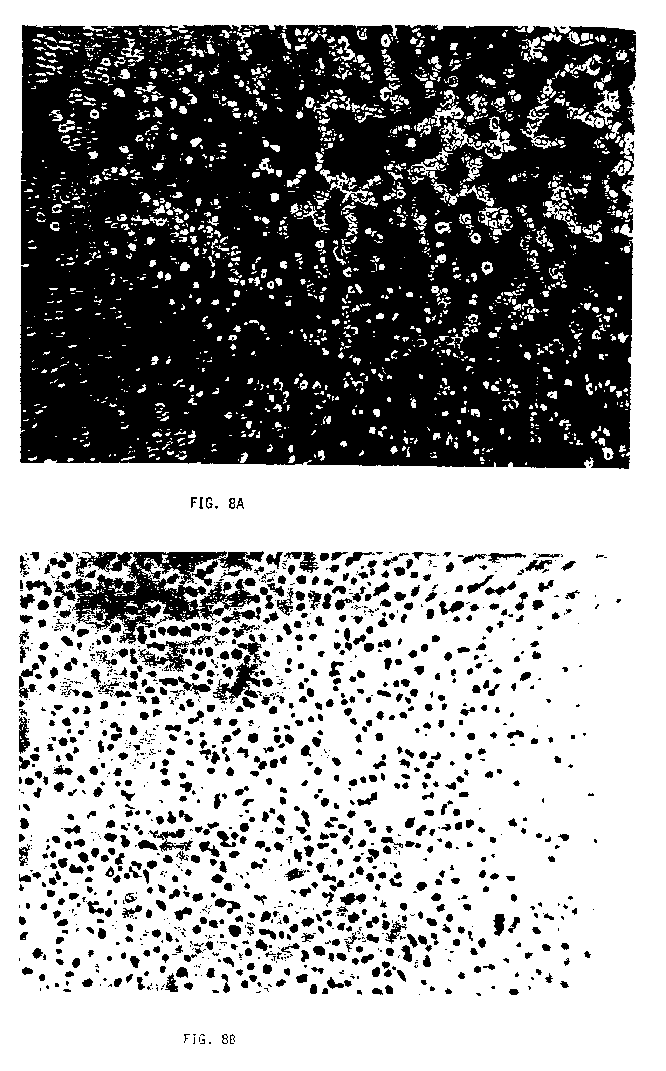

While the reconstructed membranes are resistant to treatment with mild detergents like Triton X-100, NP-40 or deoxycholate, under conditions which dissolve biological membranes and disrupt liposomes, they can be degraded by proteolytic enzymes. When haemoglobin-containing microcompartments, prepared according to Example IV, were incubated with a 0.25% trypsin solution (30 mins., 37° C), the haemoglobin was released to the surrounding medium (see Figs. 8A, 8B). This finding also points to the fact that the microcompartmentalized protein was not coagulated, but exists in a soluble form.

**Claims**

1. A semipermeable microcompartment which is artificially prepared by reassembly of proteinaceous macromolecules and which is defined by a peripheral membrane consisting substantially of a layer of said macromolecules, each of which comprises a relatively hydrophilic moiety and a relatively hydrophobic moiety and wherein the majority of such macromolecules forming the membrane are disposed with their relatively hydrophilic moities orientated outwardly from the microcompartment and their relatively hydrophobic moieties orientated inwardly towards the interior of the microcompartment.

2. A microcompartment according to claim 1 wherein substantially all of said macromolecules are disposed as aforesaid.

3. A microcompartment according to claim 1 or claim 2 which has the shape generally of a sphere.

4. A microcompartment according to claim 1 or claim 2 which has the shape generally of a partially collapsed sphere.

5. A microcompartment according to claim 1 or claim 2 which has the shape generally of a sphere inserted to fit into the central space of an annular disc.

6. A microcompartment according to any of claims 3 to 5 which has an overall diameter in the range of from about 0.1 to about 100 microns.

7. A microcompartment according to any of claims 3 to 6 which has a wall thickness in the range of from about $0.01\mu m$ (100Å) to about $0,1\mu m$ (1000Å).

8. A microcompartment according to any of the preceding claims wherein the said macromolecules comprise protein molecules.

9. A microcompartment according to any of claims 1 to 7 wherein the said macromolecules comprise glycoprotein molecules.

10. A microcompartment according to any of claims 1 to 7 wherein the said macromolecules comprise at least two of protein, glycolipid and glycoprotein molecules.

11. A microcompartment according to any of claims 8 to 10 wherein the membrane is derived from materials comprised in a naturally occurring cell membrane.

12. A microcompartment according to any of claims 8 to 10 wherein the membrane is derived from eukaryotic cells or cells of prokaryotes.

13. A microcompartment according to any of claims 8 to 10 wherein the membrane is derived from materials comprised in whole blood, red blood cell membranes, casein or egg white constituents.

14. A microcompartment according to any of claims 11 to 13 wherein the membrane contains about 90% of the proteinaceous materials in a naturally occurring cell membrane.

15. A microcompartment according to any of the preceding claims wherein the membrane encloses phospholipids.

16. A microcompartment according to any of the preceding claims wherein the membrane encloses one or more magnetic particles.

17. A microcompartment according to any of the preceding claims wherein the membrane encloses an inert bead.

18. A microcompartment according to any of claims 1 to 17 wherein the membrane encloses an antibody.

19. A microcompartment according to any of claims 1 to 17 wherein the membrane encloses a naturally-occurring or artificially produced proteinaceous substance.

20. A microcompartment according to any of claims 1 to 17 wherein the membrane encloses an enzyme or an apo-enzyme.

21. A microcompartment according to claim 20 wherein the membrane encloses an apo-enzyme together with a corresponding coenzyme.

22. A microcompartment according to any of the preceding claims wherein the membrane surface presents a specific ligand.

23. A microcompartment according to claim 22 wherein the membrane surface presents one or more substances selected from the group consisting of a hormone receptor, an antigen, an antibody and an enzyme.

24. A microcompartment according to any of the preceding claims wherein the membrane is comprised of pharmaceutically compatible materials and encloses a pharmacologically active substance.

25. A microcompartment according to claim 24 wherein the pharmacologically active substance has anti-inflammatory properties.

26. A microcompartment according to claim 25 wherein the pharmacologically active substance is a steroid.

27. A microcompartment according to claim 24 wherein the pharmacologically active substance has anticancer properties.

28. A microcompartment according to claim 27 wherein the pharmacologically active substance is cisplatin.

29. A microcompartment according to claim 27 wherein the pharmacologically active substance is doxorubicin.

30. A microcompartment according to claim 24 wherein the pharmacologically active substance has central nervous system activity.

31. A microcompartment according to claim 24 wherein the pharmacologically active substance has peripheral nervous system activity.

32. A microcompartment according to claim 24 wherein the pharmacologically active substance has analgetic activity.

33. A microcompartment according to claim 24 wherein the pharmacologically active substance has local anesthetic activity.

34. A microcompartment according to claim 24 wherein the pharmacologically active substance has narcotic activity.

35. A microcompartment according to claim 24 wherein the pharmacologically active substance has antidepressant activity.

36. A microcompartment according to claim 24 wherein the pharmacologically active substance has antibacterial activity.

37. A microcompartment according to claim 24 wherein the pharmacologically active substance has antifungal activity.

38. A microcompartment according to claim 37 wherein the pharmacologically active substance is Amphotericin B.

39. A microcompartment according to claim 24 wherein the pharmacologically active substance has antiparasitic activity.

40. A microcompartment according to claim 24 wherein the pharmacologically active substance has properties beneficial in the treatment of heart disease.

41. A microcompartment according to claim 24 wherein the pharmacologically active substance has immunomodulating activity.

42. A microcompartment according to claim 41 wherein the pharmacologically active substance has immunostimulating activity.

43. A microcompartment according to claim 24 wherein the pharmacologically active substance has immunosuppressive activity.

44. A microcompartment according to claim 41 wherein the pharmacologically active substance is a leukotriene.

45. A microcompartment according to claim 41 wherein the pharmacologically active substance is an interleukin.

46. A microcompartment according to claim 43 wherein the pharmacologically active substance is Cyclosporin A.

47. A microcompartment according to claim 24 wherein the pharmacologically active substance is a vaccine.

48. A microcompartment according to any of claims 1 to 15 wherein the membrane is composed of edible materials and encloses a foodstuff or a substance compatible therewith.

49. A microcompartment according to claim 48 wherein the membrane encloses a food flavoring substance.

50. A microcompartment according to any of claims 1 to 17 wherein the membrane encloses a substance with herbicidal, fungicidal, acaricidal or insecticidal activity.

51. A microcompartment according to any of claims 1 to 17 wherein the membrane encloses a substance with growth control activity.

52. A microcompartment according to any of claims 1 to 17 wherein the membrane encloses a pheremone.

53. A microcompartment according to any of claims 1 to 17 wherein the membrane encloses one or more hydrophobic substances.

54. A microcompartment according to any of claims 1 to 17 wherein the membrane encloses a perfume composition or perfume concentrate composition.

55. A microcompartment according to any of claims 1 to 17 wherein the membrane encloses a particulate catalyst.

56. A microcompartment according to any of claims 1 to 21 and 55 wherein the membrane encloses one or more reactant macromolecular substances of molecular weight of at least about 6000.

57. A method of effecting a chemical reaction wherein one or more reactant macromolecular substances of molecular weight of at least about 6000 which is enclosed in a microcompartment as defined in any of claims 1 to 21 and 55 is reacted in a suitable medium with a substance of molecular weight of up to about 1000.

58. A microcompartment according to claim 20 wherein the enzyme or apoenzyme is selected from the group consisting of alkaline phosphatase, asparaginase, catalase, cholesterol oxidase, cholinesterase, apo-glucoseoxidase, glucoseoxidase, peroxidase, urease, glycerolphosphate oxidase and uricase.

59. A microcompartment according to claim 58 wherein the enzyme or apo-enzyme is glucoseoxidase.

60. A process for preparing microcompartments as claimed in any of claims 1 to 14, which comprises exposing a proteinaceous macromolecular substance, which contains a relatively hydrophilic moiety and a relatively hydrophobic moiety, to the solubilization action of a homogeneous mixture of chaotropic ions in water and a dialyzable organic solvent which is not completely miscible with water, if necessary or desired subjecting the mixture to filtration or centrifugation, forming microglobules containing the microcompartment precursors, and dialyzing out the organic solvent and the chaotropic ions.

61. A process for preparing microcompartments as defined in any of claims 15 to 56, 58 and 59, which comprises exposing a proteinaceous macromolecular substance, which contains a relatively hydrophilic moiety and a relatively hydrophobic moiety, to the solubilization action of a homogeneous mixture of chaotropic ions in water and a dialyzable organic solvent which is not completely miscible with water, if necessary or desired subjecting the mixture to filtration or centrifugation, forming microglobules containing the microcompartment precursors, and dialyzing out the organic solvent and the chaotropic ion, the process being additionally characterized by the features that any soluble or insoluble substances which it is desired be enclosed by the said microcompartments are either present in the original reaction mixture or are added after the optional filtration or centrifugation step.

62. A process according to Claim 60 or Claim 61, wherein the dialysis step is effected in a dialysis bag.

63. A process according to any one of Claims 60 to 62, wherein prior to dialysis the mixture is subjected to vigorous agitation ton ensure fine division of the microglobules.

64. A process according to Claim 63 wherein the vigorous agitation is effected by ultrasonic means.

65. A process according to any one of Claims 60 to 64, wherein the organic solvent is an aliphatic alcohol containing 4, 5 or 6 carbon atoms.

66. A process according to Claim 65, wherein the aliphatic alcohol is n-butanol.

67. A process according to any one of Claims 60 to 66, wherein the chaotropic ion is $^-$SCN or $CCl_3COO^-$.

68. A process according to any one of Claims 60 to 67, using substantially equal parts by volume of the organic solvent and of an aqueous solution of chaotropic ions of concentration in the range 20 to 50% w/v.

69. A process according to any one of Claims 60 to 68, wherein, depending upon whether the organic solvent is one the water-solubility of which decreases or increase with a rise in temperature, microglobule formation is effected by respectively raising or lowering the temperature, and/or by diluting the solution with water.

70. A process for preparing microcompartments in which the peripheral fabric is formed of proteinaceous macromolecules having their relatively hydrophobic and relatively hydrophilic moieties orientated in the reverse sense from that defined in Claim 1, which comprises exposing a macromolecular substance that contains a relatively hydrophilic moiety and a relatively hydrophobic moiety to the solubilization action of a homogeneous mixture of chaotropic ions in water and a dialyzable organic solvent which is not complete miscible with water, if necessary or desired subjecting the mixture to filtration of centrifugation, forming microglobules containing the microcompartment precursors by the addition of a substantially water-immiscible organic solvent and the subsequent creation of a water-in-oil emulsion, and dialyzing out the organic solvent and the chaotropic ions into a substantially immiscible organic solvent.

**71.** A process according to Claim 70 wherein the substantially immiscible organic solvent is n-decanol.

**72.** A microcompartment produced by the process of any one of Claims 60 to 71.

**73.** A pharmaceutical composition comprising as active ingredient one or more pharmacologically active substances enclosed in microcompartments as defined in any one of Claims 24 to 47, together with a carrier or diluent.

**74.** A pharmaceutical composition according to Claim 73, wherein at least part of the carrier or diluent is enclosed in the microcompartments together with the one or more pharmacologically active substances.

**75.** A pharmaceutical composition according to Claim 73 or Claim 74, in unit dosage form.

**76.** A pharmaceutical composition according to any one of Claims 73 to 75, which is constituted in a form suitable for oral, parenteral or rectal administration, or for administration by insufflation.

**77.** A microcompartment as claimed in any one of Claims 1 to 56, 58, 59 and 72, wherein the peripheral membrane consists essentially of a monomolecular layer of said macromolecules.

**78.** A semipermeable microcompartment which is artificially prepared by reassembly of synthetic polymeric macromolecules and which is defined by a peripheral membrane consisting substantially of a layer of said macromolecules, each of which comprises a relatively hydrophilic moiety and a relatively hydrophobic moiety and wherein the majority of such macromolecules forming the membrane are disposed with their relatively hydrophilic moieties orientated outwardly from the microcompartment and their relatively hydrophobic moieties orientated inwardly towards the interior of the microcompartment.

**79.** A microcompartment according to Claim 78, wherein the said synthetic polymeric macromolecules result from the attachment of a relatively hydrophilic polymer to a relatively hydrophobic residue.

**Revendications**

**1.** Microcompartiment semiperméable, qui est préparé artificiellement par réassemblage de macromolécules protéiques et qui est défini par une membrane périphérique composée substantiellement d'une couche desdites macromolécules, dont chacune comprend un motif relativement hydrophile et un motif relativement hydrophobe, caractérisé en ce que la majorité de ces molécules, formant la membrane, sont disposées avec leur motif relativement hydrophile orienté vers l'extérieur du microcompartiment et leur motif relativement hydrophobe orienté vers l'intérieur du microcompartiment.

**2.** Microcompartiment selon la revendication 1, caractérisé en ce que pratiquement toutes lesdites macromolécules sont disposées de la manière susmentionnée.

**3.** Microcompartiment selon la revendication 1 ou 2, caractérisé en ce qu'il présente la forme générale d'une sphère.

**4.** Microcompartiment selon la revendication 1 ou 2, caractérisé on ce qu'il présente la forme générale d'une sphère en partie affaissée.

**5.** Microcompartiment selon la revendication 1 ou 2, caractérisé en ce qu'il présente la forme générale d'une sphère insérée de façon à être adaptée à l'espace central d'un disque annulaire.

**6.** Microcompartiment selon l'une quelconque des revendications 3 à 5, caractérisé en ce qu'il présente un diamètre total compris entre environ 0,1 et environ 100 μm.

**7.** Microcompartiment selon l'une quelconque des revendications 3 à 6, caractérisé en ce qu'il a une épaisseur de paroi comprise entre environ 0,01 μm (100 Å) et environ 0,1 μm (1000 Å).

**8.** Microcompartiment selon l'une quelconque des revendications précédentes, caractérisé en ce que lesdites macromolécules comprennent des molécules de protéines.

**9.** Microcompartiment selon l'une quelconque des revendications 1 à 7, caractérisé en ce que lesdites macromolécules comprennent des molécules de glycoprotéines.

**10.** Microcompartiment selon l'une quelconque des revendications 1 à 7, caractérisé en ce que lesdites macromolécules comprennent au moins deux molécules de protéines, de glycolipides et de glycoprotéines.

11. Microcompartiment selon l'une quelconque des revendications 8 à 10, caractérisé en ce que la membrane est dérivée de substances contenues dans une membrane cellulaire d'origine naturelle.

12. Microcompartiment selon l'une quelconque des revendications 8 à 10, caractérisé en ce que la membrane est dérivée de cellules eucaryotes ou de cellules procaryotes.

13. Microcompartiment selon l'une quelconque des revendications 8 à 10, caractérisé en ce que la membrane est dérivée de substances contenues dans le sang entier, dans les membranes d'hématies, dans la caséine ou dans des constituants du blanc de l'oeuf.

14. Microcompartiment selon l'une quelconque des revendications 11 à 13, caractérisé en ce que la membrane contient environ 90 % de substances protéiques d'une membrane cellulaire d'origine naturelle.

15. Microcompartiment selon l'une quelconque des revendications précédentes, caractérisé en ce que la membrane renferme des phospholipides.

16. Microcompartiment selon l'une quelconque des revendications précédentes, caractérisé en ce que la membrane renferme une ou plusieurs particules magnétiques.

17. Microcompartiment selon l'une quelconque des revendications précédentes, caractérisé en ce que la membrane renferme un bourrelet inerte.

18. Microcompartiment selon l'une quelconque des revendications 1 à 17, caractérisé en ce que la membrane renferme un anticorps.

19. Microcompartiment selon l'une quelconque des revendications 1 à 17, caractérisé en ce que la membrane renferme une substance protéique d'origine naturelle ou produite artificiellement.

20. Microcompartiment selon l'une quelconque des revendications 1 à 17, caractérisé en ce que la membrane renferme une enzyme ou une apo-enzyme.

21. Microcompartiment selon la revendication 20, caractérisé en ce que la membrane renferme une apo-enzyme en même temps qu'une co-enzyme correspondante.

22. Microcompartiment selon l'une quelconque des revendications précédentes, caractérisé en ce que la surface de la membrane présente un ligand spécifique.

23. Microcompartiment selon la revendication 22, caractérisé en ce que la surface de la membrane présente une ou plusieurs substances choisies dans le groupe comprenant un récepteur d'hormones, un antigène, un anticorps et une enzyme.

24. Microcompartiment selon l'une quelconque des revendications précédentes, caractérisé en ce que la membrane est formée de substances pharmaceutiquement compatibles et renferme une substance pharmacologiquement active.

25. Microcompartiment selon la revendication 24, caractérisé en ce que la substance pharmacologiquement active a des propriétés anti-inflammatoires.

26. Microcompartiment selon la revendication 25, caractérisé en ce que la substance pharmacologiquement active est un stéroïde.

27. Microcompartiment selon la revendication 24, caractérisé en ce que la substance pharmacologiquement active a des propriétés anticancéreuses.

28. Microcompartiment selon la revendication 27, caractérisé en ce que la substance pharmacologiquement active est le cisplatine.

29. Microcompartiment selon la revendication 27, caractérisé en ce que la substance pharmacologiquement active est la doxorubicine.

30. Microcompartiment selon la revendication 24, caractérisé en ce que la substance pharmacologiquement active a une activité sur le système nerveux central.

31. Microcompartiment selon la revendication 24, caractérisé en ce que la substance pharmacologiquement active a une activité sur le système nerveux périphérique.

32. Microcompartiment selon la revendication 24, caractérisé en ce que la substance pharmacologiquement active a une activité analgésique.

33. Microcompartiment selon la revendication 24, caractérisé en ce que la substance pharmacologiquement active a une activité anesthésique locale.

**34.** Microcompartiment selon la revendication 24, caractérisé en ce que la substance pharmacologiquement active a une activité narcotique.

**35.** Microcompartiment selon la revendication 24, caractérisé en ce que la substance pharmacologiquement active a une activité antidépressive.

**36.** Microcompartiment selon la revendication 24, caractérisé en ce que la substance pharmacologiquement active a une activité antibactérienne.

**37.** Microcompartiment selon la revendication 24, caractérisé en ce que la substance pharmacologiquement active a une activité antifongique.

**38.** Microcompartiment selon la revendication 37, caractérisé en ce que la substance pharmacologiquement active est l'amphotéricine B.

**39.** Microcompartiment selon la revendication 24, caractérisé en ce que la substance pharmacologiquement active a une activité antiparasitaire.

**40.** Microcompartiment selon la revendication 24, caractérisé en ce que la substance pharmacologiquement active a des propriétés qui sont bénéfiques dans le traitement des maladies cardiaques.

**41.** Microcompartiment selon la revendication 24, caractérisé en ce que la substance pharmacologiquement active a une activité immunorégulatrice.

**42.** Microcompartiment selon la revendication 41, caractérisé en ce que la substance pharmacologiquement active a une activité immunostimulante.

**43.** Microcompartiment selon la revendication 24, caractérisé en ce que la substance pharmacologiquement active a une activité immunodépressive.

**44.** Microcompartiment selon la revendication 41, caractérisé on ce que la substance pharmacologiquement active est une leucotriène.

**45.** Microcompartiment selon la revendication 41, caractérisé en ce que la substance pharmacologiquement active est une interleukine.

**46.** Microcompartiment selon la revendication 43, caractérisé en ce que la substance pharmacologiquement active est une cyclosporine A.

**47.** Microcompartiment selon la revendication 24, caractérisé en ce que la substance pharmacologiquement active est un vaccin.

**48.** Microcompartiment selon l'une quelconque des revendications 1 à 15, caractérisé en ce que la membrane est composée de substances comestibles et renferme un aliment ou une substance compatible avec lesdites substances.

**49.** Microcompartiment selon la revendication 48, caractérisé en ce que la membrane renferme une substance alimentaire aromatisante.

**50.** Microcompartiment selon l'une quelconque des revendications 1 à 17, caractérisé en ce que la membrane renferme une substance ayant une activité herbicide, fongicide, acaricide ou insecticide.

**51.** Microcompartiment selon l'une quelconque des revendications 1 à 17, caractérisé en ce que la membrane renferme une substance ayant une activité de régulation de la croissance.

**52.** Microcompartiment selon l'une quelconque des revendications 1 à 17, caractérisé en ce que la membrane renferme une phéromone.

**53.** Microcompartiment selon l'une quelconque des revendications 1 à 17, caractérisé en ce que la membrane renferme une ou plusieurs substances hydrophobes.

**54.** Microcompartiment selon l'une quelconque des revendications 1 à 17, caractérisé en ce que la membrane renferme une composition à base de parfum ou une composition à base de concentré de parfum.

**55.** Microcompartiment selon l'une quelconque des revendications 1 à 17, caractérisé en ce que la membrane renferme un catalyseur particulaire.

**56.** Microcompartiment selon l'une quelconque des revendications 1 à 21 et 55, caractérisé en ce que la membrane renferme une ou plusieurs substances macromoléculaires capables de réagir, d'un poids moléculaire d'au moins environ 6.000.

**57.** Procédé de réalisation d'une réaction chimique, caractérisé en ce qu'une ou plusieurs

substances macromolécculaires réactives, d'un poids moléculaire d'au moins environ 6.000, comprise(s) dans un microcompartiment selon l'une quelconque des revendications 1 à 21 et 55, est/sont mise(s) en réaction dans un milieu approprié avec une substance d'un poids moléculaire allant jusqu'à environ 1.000.

58. Microcompartiment selon la revendication 20, caractérisé en ce que l'enzyme ou l'apo-enzyme est choisie dans le groupe comprenant la phosphatase alcaline, l'asparaginase, la catalase, la cholestérol-oxydase, la cholestérol-estérase, l'apoglucose-oxydase, la glucose-oxydase, la peroxydase, l'uréase, la glycérolphosphate-oxydase et l'uricase.

59. Microcompartiment selon la revendication 58, caractérisé en ce que l'enzyme ou l'apo-enzyme est la glucose-oxydase.

60. Procédé de préparation de microcompartiments selon l'une quelconque des revendications 1 à 14, caractérisé en ce qu'il comprend les étapes consistant à soumettre une substance protéique macromoléculaire, qui comporte un motif relativement hydrophile et un motif relativement hydrophobe, à l'action de solubilisation d'un mélange homogène d'ions chaotropiques dans de l'eau et d'un solvant organique dialysable qui n'est pas complètement miscible avec l'eau, si nécessaire, ou si on le souhaite, soumettre le mélange à une filtration ou à une centrifugation, former des microglobules contenant les précurseurs du microcompartiment, et éliminer le solvant organique et les ions chaotropiques par dialyse.

61. Procédé de préparation de microcompartiments selon l'une quelconque des revendications 15 à 56, 58 et 59, caractérisé en ce qu'il comprend les étapes consistant à soumettre une substance protéique macromoléculaire, qui comporte un motif relativement hydrophile et un motif relativement hydrophobe, à l'action de solubilisation d'un mélange homogène d'ions chaotropiques dans de l'eau et d'un solvant organique dialysable qui n'est pas complètement miscible avec l'eau, si nécessaire, ou si on le souhaite, soumettre le mélange à une filtration ou à une centrifugation, former des microglobules contenant les précurseurs du microcompartiment, et éliminer le solvant organique et les ions chaotropiques par dialyse, le procédé étant caractérisé en outre en ce que n'importe quelles substances solubles ou insolubles qu'on souhaite inclure dans lesdits microcompartiments sont contenues dans le mélange réactionnel d'origine ou on les ajoute après l'éventuelle étape de filtration ou de centrifugation.

62. Procédé selon la revendication 60 ou 61, caractérisé en ce que l'étape de dialyse est réalisée dans un sac à dialyse.

63. Procédé selon l'une quelconque des revendications 60 à 62, caractérisé en ce qu'on soumet le mélange, avant la dialyse, à une agitation énergique pour garantir une fine division des microglobules.

64. Procédé selon la revendication 63, caractérisé en ce qu'on effectue l'agitation énergique par des moyens à ultrasons.

65. Procédé selon l'une quelconque des revendications 60 à 64, caractérisé en ce que le solvant organique est un alcool aliphatique comportant 4, 5 ou 6 atomes de carbone.

66. Procédé selon la revendication 65, caractérisé en ce que l'alcool aliphatique est du n-butanol.

67. Procédé selon l'une quelconque des revendications 60 à 66, caractérisé en ce que l'ion chaotropique est le $^-SCN$ ou le $CCl_3COO^-$.

68. Procédé selon l'une quelconque des revendications 60 à 67, caractérisé en ce qu'on utilise des parties en volume substantiellement égales du solvant organique et d'une solution aqueuse d'ions chaotropiques à une concentration comprise entre 20 et 50 % p/v.

69. Procédé selon l'une quelconque des revendications 60 à 68, caractérisé en ce que, selon que le solvant organique est un solvant dont la solubilité dans l'eau augmente ou diminue lorsque la température augmente, on réalise la formation des microglobules respectivement en augmentant ou en abaissant la température, et/ou en diluant la solution avec de l'eau.

70. Procédé de préparation de microcompartiments dans lesquels le tissu périphérique est formé de macromolécules protéiques ayant leur motif relativement hydrophobe et leur motif relativement hydrophile orientés dans le sens inverse à celui défini dans la revendication 1, caractérisé en ce qu'il comprend les étapes consistant à soumettre une substance macromoléculaire, qui contient un motif relativement hydrophile et un motif relativement hydrophobe, à l'action de solubilisation d'un mélange homogène d'ions chaotropiques dans de l'eau et d'un solvant organique dialysable qui n'est pas complètement miscible avec l'eau, si nécessaire ou si on le souhaite, soumettre le mélange à une filtration ou à une centrifugation, former des microglobules contenant les précurseurs du microcompartiment, par addition d'un solvant organique substantiellement non miscible avec l'eau et par formation subséquente d'une émulsion eau-dans-huile, et éliminer par dialyse le solvant organique et les ions chaotropiques dans un solvant organique substantiellement non miscible avec l'eau.

71. Procédé selon la revendication 70, caractérisé en ce que le solvant organique substantiellement non miscible est le n-décanol.

72. Microcompartiment préparé au moyen du procédé selon l'une quelconque des revendications 60 à 71.

73. Composition pharmaceutique comprenant, comme ingrédient actif, une ou plusieurs substances pharmacologiquement actives qui sont contenues dans des microcompartiments selon l'une quelconque des revendications 24 à 47, conjointement avec un excipient ou un diluant.

74. Composition pharmaceutique selon la revendication 73, caractérisée en ce qu'au moins une partie de l'excipient ou du diluant est contenue dans les microcompartiments, conjointement avec la ou les substances pharmacologiquement actives.

75. Composition pharmaceutique selon la revendication 73 ou 74, caractérisée en ce qu'elle se présente sous forme de doses unitaires.

76. Composition pharmaceutique selon l'une quelconque des revendications 73 à 75, caractérisée en ce qu'elle se présente sous une forme appropriée à une administration par voie buccale, parentérale ou rectale, ou à une administration par insufflation.

77. Microcompartiment selon l'une quelconque des revendications 1 à 56, 58, 59 et 72, caractérisé en ce que la membrane périphérique est formée essentiellement d'une couche monomoléculaire desdites macromolécules.

78. Microcompartiment semiperméable, qui est préparé artificiellement par réassemblage de macromolécules synthétiques polymères et qui est défini par une membrane périphérique composée substantiellement d'une couche desdites macromolécules, dont chacune comprend un motif relativement hydrophile et un motif relativement hydrophobe, caractérisé en ce que la plupart de ces molécules, formant la membrane, sont disposées avec leur motif relativement hydrophile orienté vers l'extérieur du microcompartiment et leur motif relativement hydrophobe orienté vers l'intérieur du microcompartiment.

79. Microcompartiment selon la revendication 78, caractérisé en ce que lesdites macromolécules synthétiques polymères résultent de la fixation d'un polymère relativement hydrophile sur un résidu relativement hydrophobe.

## Patentansprüche

1. Semipermeable Mikrozelle, die künstlich durch Zusammenbau von proteinartigen Makromolekülen hergestellt wurde und die durch eine periphere Membran, welche im wesentlichen aus einer Schicht dieser Makromoleküle besteht, definiert ist, von denen jedes einen relativ hydrophilen Rest und einen relativ hydrophoben Rest umfaßt und bei denen die Mehrheit dieser Makromoleküle, die die Membran bilden, mit ihren relativ hydrophilen Resten von der Mikrozelle nach außen orientiert und mit ihren relativ hydrophoben Resten zum Inneren der Mikrozelle nach innen orientiert angeordnet ist.

2. Mikrozelle nach Anspruch 1, bei der im wesentlichen alle der Makromoleküle wie oben angeordnet sind.

3. Mikrozelle nach Anspruch 1 oder Anspruch 2, die die allgemeine Form einer Kugel hat.

4. Mikrozelle nach Anspruch 1 oder Anspruch 2, die die allgemeine Form einer teilweise zusammengefallenen Kugel hat.

5. Mikrozelle nach Anspruch 1 oder Anspruch 2, die die allgemeine Form einer eingesetzten Kugel hat, die in den mittigen Raum einer Ringscheibe paßt.

6. Mikrozelle nach einem der Ansprüche 3 bis 5, die einen Gesamtdurchmesser im Bereich von etwa 0,1 bis etwa 100 Mikron hat.

7. Mikrozelle nach einem der Ansprüche 3 bis 6, die eine Wanddicke im Bereich von etwa 0,01 $\mu$m (100 Å) bis etwa 0,1 $\mu$m (1000 Å) hat.

8. Mikrozelle nach einem der vorausgehenden Ansprüche, bei der die Makromoleküle Proteinmoleküle umfassen.

9. Mikrozelle nach einem der Ansprüche 1 bis 7, bei der die Makromoleküle Glycoproteinmoleküle umfassen.

10. Mikrozelle nach einem der Ansprüche 1 bis 7, bei der die Makromoleküle wenigstens zwei Arten von Protein-, Glycolipid- und Glycoproteinmolekülen umfassen.

11. Mikrozelle nach einem der Ansprüche 8 bis 10, bei der Membran sich von Materialien herleitet, die in einer natürlich vorkommenden Zellmembran enthalten sind.

12. Mikrozelle nach einem der Ansprüche 8 bis 10, bei der die Membran sich von eukaryontischen Zellen oder Prokaryontenzellen herleitet.

13. Mikrozelle nach einem der Ansprüche 8 bis 10, bei der die Membran sich von Materialien herleitet, die in Gesamtblut, roten Blutzellmembranen, Casein oder Eiweißbestandteilen enthalten sind.

14. Mikrozelle nach einem der Ansprüche 11 bis 13, bei der die Membran etwa 90 % der proteinartigen Materialien in einer natürlich vorkommenden Zellmembran enthält.

15. Mikrozelle nach einem der vorausgehenden Ansprüche, bei der die Membran Phospholipide einschließt.

16. Mikrozelle nach einem der vorausgehenden Ansprüche, bei der die Membran ein oder mehrere magnetische Teilchen einschließt.

17. Mikrozelle nach einem der vorausgehenden Ansprüche, bei der die Membran ein inertes Külgelchen einschließt.

18. Mikrozelle nach einem der Ansprüche 1 bis 17, bei der die Membran einen Antikörper einschließt.

19. Mikrozelle nach einem der Ansprüche 1 bis 17, bei der die Membran eine natürlich vorkommende oder künstlich hergestellte proteinartige Substanz einschließt.

20. Mikrozelle nach einem der Ansprüche 1 bis 17, bei der die Membran ein Enzym oder ein Apoenzym einschließt.

21. Mikrozelle nach Anspruch 20, bei der die Membran ein Apoenzym zusammen mit einem entsprechenden Coenzym einschließt.

22. Mikrozelle nach einem der vorausgehenden Ansprüche, bei der die Membranoberfläche einen spezifischen Liganden aufweist.

23. Mikrozelle nach Anspruch 22, bei der die Membranoberfläche ein oder mehrere Substanzen aus der Gruppe eines Hormonrezeptors, eines Antigens, eines Antikörpers und eines Enzyms aufweist.

24. Mikrozelle nach einem der vorausgehenden Ansprüche, bei der die Membran pharmazeutisch verträgliche Materialien umfaßt und eine pharmakologisch wirksame Substanz einschließt.

25. Mikrozelle nach Anspruch 24, bei der die pharmakologisch wirksame Substanz enzündungshemmende Eigenschaften hat.

26. Mikrozelle nach Anspruch 25, bei der die pharmakologisch wirksame Substanz ein Steroid ist.

27. Mikrozelle nach Anspruch 24, bei der die pharmakologisch wirksame Substanz Anticancereigenschaften hat.

28. Mikrozelle nach Anspruch 27, bei der die pharmakologisch wirksame Substanz Cisplatin ist.

29. Mikrozelle nach Anspruch 27, bei der die pharmakologisch wirksame Substanz Doxorubicin ist.

30. Mikrozelle nach Anspruch 24, bei der die pharmakologisch wirksame Substanz Wirksamkeit auf das zentrale Nervensystem hat.

31. Mikrozelle nach Anspruch 24, bei der die pharmakologisch wirksame Substanz Wirksamkeit auf das periphere Nervensystem hat.

32. Mikrozelle nach Anspruch 24, bei der die pharmakologisch wirksame Substanz analgetische Wirksamkeit hat.

33. Mikrozelle nach Anspruch 24, bei der die pharmakologisch wirksame Substanz lokalanesthetische Wirksamkeit hat.

34. Mikrozelle nach Anspruch 24, bei der die pharmakologisch wirksame Substanz narkotische Wirksamkeit hat.

35. Mikrozelle nach Anspruch 24, bei der die pharmakologisch wirksame Substanz antidepressive Wirksamkeit hat.

36. Mikrozelle nach Anspruch 24, bei der die pharmakologisch wirksame Substanz antibakterielle Wirksamkeit hat.

37. Mikrozelle nach Anspruch 24, bei der die pharmakologisch wirksame Substanz Wirksamkeit gegen Pilze hat.

38. Mikrozelle nach Anspruch 37, bei der die pharmakologisch wirksame Substanz Amphotericin B ist.

39. Mikrozelle nach Anspruch 24, bei der die pharmakologisch wirksame Substanz Wirksamkeit gegen Parasiten hat.

40. Mikrozelle nach Anspruch 24, bei der die pharmakologisch wirksame Substanz günstige Eigenschaften bei der Behandlung von Herzerkrankungen hat.

41. Mikrozelle nach Anspruch 24, bei der die pharmakologisch wirksame Substanz immunmodulierende Wirksamkeit hat.

42. Mikrozelle nach Anspruch 41, bei der die pharmakologisch wirksame Substanz immunstimulierende Wirksamkeit hat.

43. Mikrozelle nach Anspruch 24, bei der die pharmakologisch wirksame Substanz immunsuppressive Wirksamkeit hat.

44. Mikrozelle nach Anspruch 41, bei der die pharmakologisch wirksame Substanz Leukotrien ist.

45. Mikrozelle nach Anspruch 41, bei der die pharmakologisch aktive Substanz Interleukin ist.

46. Mikrozelle nach Anspruch 43, bei der die pharmakologisch wirksame Substanz Cyclosporin A ist.

47. Mikrozelle nach Anspruch 24, bei der die pharmakologisch wirksame Substanz ein Vaccin ist.

48. Mikrozelle nach einem der Ansprüche 1 bis 15, bei der die Membran aus eßbaren Materialien aufgebaut ist und ein Nahrungsmittel oder eine damit verträgliche Substanz einschließt.

49. Mikrozelle nach Anspruch 48, bei der die Membran einen Nahrungsmittelgeschmacksstoff einschließt.

50. Mikrozelle nach einem der Ansprüche 1 bis 17, bei der die Membran eine Substanz mit herbizider, fungizider, acarizider oder insectizider Wirksamkeit einschließt.

51. Mikrozelle nach einem der Ansprüche 1 bis 17, bei der die Membran eine Substanz mit wachstumssteuernder Wirksamkeit einschließt.

52. Mikrozelle nach einem der Ansprüche 1 bis 17, bei der die Membran ein Pheremon einschließt.

53. Mikrozelle nach einem der Ansprüche 1 bis 17, bei der die Membran eine oder mehrere hydrophobe Substanzen einschließt.

54. Mikrozelle nach einem der Ansprüche 1 bis 17, bei der die Membran eine Parfumzusammensetzung oder Parfumkonzentratzusammensetzung einschließt.

55. Mikrozelle nach einem der Ansprüche 1 bis 17, bei der die Membran einen feinteiligen Katalysator einschließt.

56. Mikrozelle nach einem der Ansprüche 1 bis 21 und 55, bei der die Membran eine oder mehrere reaktive makromolekulare Substanzen mit einem Molekulargewicht von wenigstens etwa 6000 einschließt.

57. Verfahren zur Durchführung einer chemischen Reaktion, bei der eine oder mehrere reaktive makromolekulare Substanzen mit einem Molekulargewicht von wenigstens etwa 6000, die in einer Mikrozelle gemäß einem der Ansprüche 1 bis 21 und 55 eingeschlossen sind, in einem geeigneten Medium mit einer Substanz mit einem Molekulargewicht von bis zu etwa 1000 umgesetzt werden.

58. Mikrozelle nach Anspruch 20, bei der das Enzym oder Apoenzym aus der Gruppe alkalische Phosphatase, Asparaginase, Katalase, Cholesterinoxidase, Cholesterase, Apoglucoseoxidase, Glucoseoxidase, Peroxidase, Urease, Glycerinphosphatoxidase und Uricase ausgewählt ist.

59. Mikrozelle nach Anspruch 58, bei der das Enzym oder Apoenzym Glucoseoxidase ist.

60. Verfahren zur Herstellung von Mikrozellen nach einem der Ansprüche 1 bis 14, bei dem man eine proteinartige makromolekulare Substanz, die einen relativ hydrophilen Rest und einen relativ hydrophoben Rest enthält, der löslich machenden Wirkung eines homogenen Gemisches von chaotropen Ionen in Wasser und eines dialysierbaren organischen Lösungsmittels, das mit Wasser nicht vollständig mischbar ist, aussetzt, erforderlichenfalls oder erwünschtenfalls das Gemisch filtriert oder zentrifugiert, die Mikrozellenvorläufer enthaltende Mikrokügelchen bildet und das organische Lösungsmittel und die chaotropen Ionen ausdialysiert.

61. Verfahren zur Herstellung von Mikrozellen nach einem der Ansprüche 15 bis 56, 58 und 59, bei dem man eine proteinartige makromolekulare Substanz, die einen relativ hydrophilen Rest und einen relativ hydrophoben Rest enthält, der löslich machenden Wirkung eines homogenen Gemisches chaotroper Ionen in Wasser und eines dialysierbaren organischen Lösungsmittels, das mit Wasser nicht vollständig mischbar ist, aussetzt, erforderlichenfalls oder erwünschtenfalls das Gemisch filtriert oder zentrifugiert, die Mikrozellenvorläufer enthaltende Mikrokügelchen bildet und das organische Lösungsmittel und das chaotrope Ion ausdialysiert, wobei das Verfahren zusätzlich durch die Merkmale gekennzeichnet ist, daß lösliche oder unlösliche Substanzen, die gegebenenfalls von den Mikrozellen eingeschlossen sind, entweder in dem ursprünglichen Reaktionsgemisch vorhanden sind oder nach der gegebenenfalls erfolgenden Filtrations- oder Zentrigierstufe zugegeben werden.

62. Verfahren nach Anspruch 60 oder Anspruch 61, bei dem die Dialysestufe in einem Dialysebeutel durchgeführt wird.

63. Verfahren nach einem der Ansprüche 60 bis 62, bei dem vor der Dialyse das Gemisch heftigem Rühren unterzogen wird, um eine feine Aufteilung der Mikrokügelchen zu gewährleisten.

64. Verfahren nach Anspruch 63, bei dem das heftige Röhren durch eine Ultraschalleinrichtung bewirkt wird.

65. Verfahren nach einem der Ansprüche 60 bis 64, bei dem das organische Lösungsmittel ein aliphatischer Alkohol mit 4, 5 oder 6 Kohlenstoffatomen ist.

66. Verfahren nach Anspruch 65, bei dem der aliphatische Alkohol n-Butanol ist.

67. Verfahren nach einem der Ansprüche 60 bis 66, bei dem das chaotrope Ion $^-$SCN oder $CCl_3COO^-$ ist.

68. Verfahren nach einem der Ansprüche 60 bis 67 unter Verwendung im wesentlichen gleicher Volumenteile des organischen Lösungsmittels und einer wäßrigen Lösung chaotroper Ionen einer Konzentration im Bereich von 20 bis 50 Gewichts/Volumen-%.

69. Verfahren nach einem der Ansprüche 60 bis 68, bei dem je nachdem, ob das organische Lösungsmittel ein solches ist, dessen Wasserlöslichkeit mit einem Temperaturanstieg fällt oder steigt, die Mikrokügelchenbildung durch Steigerung oder Senkung der Temperatur und/oder durch Verdünnen der Lösung mit Wasser erfolgt.

70. Verfahren zur Herstellung von Mikrozellen, bei dem die periphere Membran von proteinartigen Makromolekülen gebildet wird, die ihre relativ hydrophoben und relativ hydrophilen Reste in umgekehrtem Sinne von jenem gemäß Anspruch 1 orientiert haben, indem man eine makromolekulare Substanz, die einen relativ hydrophilen Rest und einen relativ hydrophoben Rest enthält, der löslich machenden Wirkung eines homogenen Gemisches chaotroper Ionen in Wasser und eines dialysierbaren organischen Lösungsmittels, das mit Wasser nicht vollständig mischbar ist, aussetzt, erforderlichenfalls oder erwünschtenfalls das Gemisch filtriert oder zentrifugiert, die Mikrozellenvorläufer enthaltende Mikrokügelchen durch Zugabe eines im wesentlichen wasserunmischbaren organischen Lösungsmittels und die daraus folgernde Erzeugung einer Wasser-in-Öl-Emulsion bildet und das organische Lösungsmittel und die chaotropen Ionen in ein im wesentlichen unmischbares organisches Lösungsmittel ausdialysiert.

71. Verfahren nach Anspruch 70, bei dem das im wesentlichen unmischbare organische Lösungsmittel n-Decanol ist.

72. Mikrozelle, die nach dem Verfahren nach einem der Ansprüche 60 bis 71 hergestellt ist.

73. Pharmazeutische Zusammensetzung, die als wirksamen Bestandteil eine oder mehrere pharmakologisch wirksame Substanzen, die in Mikrozellen nach einem der Ansprüche 24 bis 47 eingeschlossen sind, zusammen mit einem Trägermaterial oder Verdünnungsmittel aufweist.

74. Pharmazeutische Zusammensetzung nach Anspruch 73, bei der wenigstens ein Teil des Trägermaterials oder Verdünnungsmittels zusammen mit der oder den pharmakologisch wirksamen Substanzen in den Mikrozellen eingeschlossen ist.

75. Pharmazeutische Zusammensetzung nach Anspruch 73 oder Anspruch 74 in Einheitsdosierungsform.

76. Pharmazeutische Zusammensetzung nach einem der Ansprüche 73 bis 75, die in einer für orale, parenterale oder rectale Verabreichung oder für Verabreichung durch Insufflation geeigneten Form vorliegt.

77. Mikrozelle nach einem der Ansprüche 1 bis 56, 58, 59 und 72, bei der die pheriphere Membran im wesentlichen aus einer monomolekularen Schicht der Makromoleküle besteht.

78. Semipermeable Mikrozelle, die künstlich durch Zusammenbau synthetischer polymerer Makromoleküle hergestellt ist und die durch eine periphere Membran definiert ist, die im wesentlichen aus einer Schicht dieser Makromoleküle besteht, von denen jedes einen relativ hydrophilen Rest und einen relativ hydrophoben Rest besitzt und worin die Mehrheit dieser Makromoleküle, die die Membran bilden, mit ihren relativ hydrophilen Resten von der Mikrozelle nach außen orientiert und mit ihren relativ hydrophoben Resten zum Inneren der Mikrozelle nach innen orientiert angeordnet ist.

79. Mikrozelle nach Anspruch 78, bei der die synthetischen polymeren Makromoleküle aus der Bindung eines relativ hydrophilen Polymers an einen relativ hydrophoben Rest resultieren.

FIG. 1

FIG. 2A

FIG. 2B

FIG. 3A

FIG. 3B

FIG. 3C

FIG. 3D

FIG. 4A

FIG. 4B

FIG. 5A

FIG. 5B

FIG. 6

Fig. 7

FIG. 8A

FIG. 8B